(19) Europäisches Patentamt / European Patent Office / Office européen des brevets

(11) **EP 2 098 363 B1**

(12) **EUROPEAN PATENT SPECIFICATION**

(45) Date of publication and mention
of the grant of the patent:
**30.10.2013 Bulletin 2013/44**

(51) Int Cl.:
*A61F 13/49* (2006.01)   *A61F 13/511* (2006.01)
*A61F 13/514* (2006.01)   *B32B 25/08* (2006.01)
*B32B 27/02* (2006.01)   *B32B 27/06* (2006.01)
*B32B 27/08* (2006.01)   *B32B 27/12* (2006.01)
*B32B 27/28* (2006.01)   *B32B 27/32* (2006.01)
*B32B 27/36* (2006.01)   *B32B 5/08* (2006.01)
*B32B 5/26* (2006.01)   *B32B 7/14* (2006.01)
*B32B 25/10* (2006.01)   *B32B 25/14* (2006.01)

(21) Application number: **07832596.6**

(22) Date of filing: **27.11.2007**

(86) International application number:
**PCT/JP2007/072871**

(87) International publication number:
**WO 2008/081662 (10.07.2008 Gazette 2008/28)**

(54) **STRETCH SHEET AND PROCESS OF PRODUCING THE SAME**

DEHNBARE FOLIE UND VERFAHREN ZUR HERSTELLUNG DER DEHNBAREN FOLIE

FEUILLE ÉTIRABLE ET PROCÉDÉ DE PRODUCTION DE FEUILLE ÉTIRABLE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.12.2006   JP 2006356560**
**24.07.2007   JP 2007192665**
**07.09.2007   JP 2007233410**

(43) Date of publication of application:
**09.09.2009 Bulletin 2009/37**

(73) Proprietor: **Kao Corporation**
**Chuo-ku**
**Tokyo 103-8210 (JP)**

(72) Inventors:
• **MIYAMOTO, Takanobu**
**Haga-gun**
**Tochigi 321-3497 (JP)**
• **KOBAYASHI, Hideyuki**
**Haga-gun**
**Tochigi 321-3497 (JP)**
• **KANAZAWA, Koji**
**Haga-gun**
**Tochigi 321-3497 (JP)**
• **KOHIRA, Hiroshi**
**Haga-gun**
**Tochigi 321-3497 (JP)**
• **SAKA, Wataru**
**Haga-gun**
**Tochigi 321-3497 (JP)**
• **MIYAMURA, Takeshi**
**Haga-gun**
**Tochigi 321-3497 (JP)**

(74) Representative: **Vossius & Partner**
**Siebertstrasse 4**
**81675 München (DE)**

(56) References cited:
WO-A1-99/17926        WO-A1-03/017898
WO-A1-2005/065932     JP-A- 04 030 847
JP-A- 08 300 436      JP-A- 2002 283 479
JP-A- 2005 514 232    JP-A- 2005 514 244
JP-A- 2006 520 701

**Description**

Technical Field

The present invention relates to a stretch sheet composed of elastic filaments and a nonwoven fabric and a process of producing the same.

Background Art

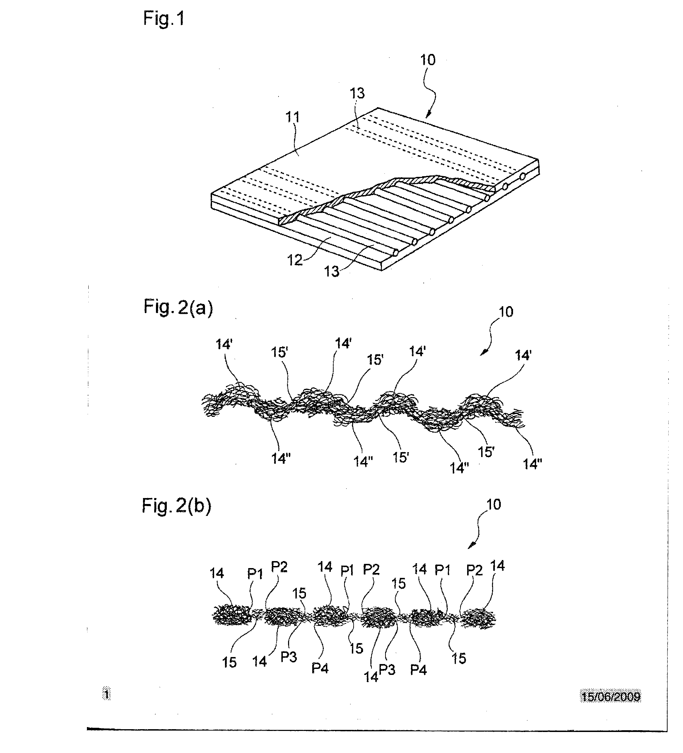

Conventional techniques pertinent to a composite stretch sheet composed of elastic fibers and a nonwoven fabric include those disclosed in patent document 1 and patent document 2. The stretch sheets according to these references are composed of, as illustrated in Fig. 1 of the respective documents, a first nonwoven fabric layer 122, a second nonwoven fabric layer 126, and an elastomer layer 124 interposed between the two nonwoven fabrics. The elastomer layer 124 is formed of scrim, perforated film, elastomer woven or nonwoven. Figs. 1 of the documents 1 and 2 show that the elastomer layer 124 is formed of an elastomer scrim 130 made of first strands 125 and second strands 127 perpendicular to the first strands 125. Because of this structure of the elastomer layer 124, when the stretch sheets of patent documents 1 and 2 are stretched in a direction, they undergo contraction in the direction perpendicular to the stretch direction. Where they are used as, for example, an outer cover of a pull-on diaper, the outer cover stretches in the body circumferential direction but contracts in the diaper longitudinal direction (this phenomenon will hereinafter be referred to as contraction in width or width contraction), which causes the diaper to slide down or wrinkle. Considering the structure of a diaper and the motion of a wearer, a stretchable diaper experiences non-uniform stretch around the circumferential direction. This makes the width contraction more conspicuous. Because the strands 125 and 127 and the nonwoven fabric layers 122 and 126 are bonded by heat and pressure, the strands 125 and 127 bite into the nonwoven fabric layers 122 and 126 to impair the soft and fluffy feel of the nonwoven fabrics 122 and 126. Since the heat and pressure for bonding the strands 125 and 127 and the nonwoven fabric layers 122 and 126 are applied from the side of the nonwoven fabric layers 122 or 126, the outer nonwoven fabric side is liable to have a higher temperature, while the inner strand side temperature is liable to have a lower temperature. In an attempt to obtain a sufficient bonding strength, there is a tendency that the nonwoven fabric is fused into a cohesive film-like layer before the strands are. The inside fibers of the nonwoven fabrics in contact with the strands must be fused to achieve high bonding strength, so that a higher temperature or a higher pressure should be applied, which further deteriorates the feel of the nonwoven fabrics. Fusion of the fibers of the nonwoven fabrics may be avoided by using high melting nonwoven fabrics, but such nonwoven fabrics are hard to be fusion bonded with elastomer scrim only to provide a composite sheet with reduced peel strength. Additionally, it is difficult with high melting nonwoven fabrics to obtain a composite sheet with a soft feel.

The technique of patent document 3 is also known for the production of a stretch sheet. According to Figs. 1, 2, and 7 of patent document 3, the elastic sheet-like composite (stretch sheet) is composed of a sheet 12 having a large number of arcuate portions 13 formed by using corrugating members 20 and 21 and elastic strands 16 fusion bonded to the tops (or bottoms) of the arcuate portions 13. The elastic strands 16 are melt extruded from a die 22 and fusion bonded to the sheet 12 in their unstretched state. Therefore, the elastic strands 16 and the sheet 12 are bonded by point contact. Because of this, it is not easy to increase the bonding strength. The stretch sheet disclosed is unable to stretch beyond its fully straightened out length. Forming the sheet 12 to have the arcuate portions 13 involves lavish use of the sheet 12, which is not only uneconomical but reduces breathability. Patent document 3 mentions that a web of the sheet 12 may be made extensible before being joined with the elastic strands 16. It is difficult, nevertheless, to transport a lengthwise extensible nonwoven fabric web without allowing the web to extend. Furthermore, the stretch sheet of patent document 3 has the problem of storage when it is stored in roll form. That is, stress relaxation occurs; that is, the contractibility gradually reduces while the composite sheet is kept under take-up tension during long-term storage in roll form. In cases where the stretch sheet is subjected to any post-processing operation in a different site, it is inevitable to take-up the stretch sheet in a roll, taking ease of handling and transport into consideration.

The elastic sheet-like composite of patent document 3 includes a sheet, such as a nonwoven fabric, and elastic strands extending in one direction without intersecting with each other. The elastic strands are made from a molten thermoplastic material which becomes resiliently elastic when cooled (e.g., elastomeric polyester, polyurethane, polystyrene-polyisoprene-polystyrene,

polystyrene-polybutadiene-polystyrene, or

polystyrene-poly(ethylene-butylene) -polystyrene). Such a stretch sheet having filaments extending in one direction in a non-intersection relationship is produced by extruding molten filaments from spinning nozzles spaced in the direction perpendicular to the moving direction of a sheet web and fusion bonding the molten filaments to the sheet web.

However, the mentioned method of making a stretch sheet has a problem that the molten filaments extruded from the spinning nozzles tend to vibrate due to airflow, static electricity, or other causes before being fusion bonded to the sheet. As a result, the vibrating filaments may be bonded to the sheet as such, resulting in a failure to provide a stretch sheet having the filaments extending straight in an equally spaced parallel relationship. Although the filaments extruded from the spinning nozzles could be prevented from vibrating by increasing the take-off tension, applying a tension enough to prevent vibration of molten filaments can cause breakage of the filaments. Breakage of filaments during take-off often occurs particularly with fine filaments and has posed a serious problem of reduction in productivity of a stretch sheet. On the other hand, elastic yarns finer than those used in currently commercially available absorbent articles, such as diapers, have been desired to provide a stretch sheet with further improved feel.

Patent document 4 teaches a method of making a stretch sheet including the steps of forming elastic strands, drawing the elastic strands in their cooled and solidified state, and joining the drawn strands with a nonwoven fabric. Upon the elastic strands retracting, the nonwoven fabric gathers or is slackened, thereby to provide the nonwoven fabric with stretchability within the length of slack. Since the elastic strands have been cool-solidified when they are bonded to nonwoven fabric, an adhesive is used to bond the strands and the nonwoven fabric. An increased amount of an adhesive will be necessary to obtain sufficient bonding strength. A large amount of an adhesive will fill the interstices between fibers of the nonwoven fabric, resulting in reduction of breathability. An adhesive will also bind a plurality of the fibers, resulting in an increased stiffness. It is therefore difficult to obtain a soft and breathable sheet. Since the stretch sheet of patent document 4 has the same structure as an ordinary, rubber thread-elasticized sheet composed of elastic threads bonded in their stretched state, it gathers in its relaxed state, having poor feel to the touch and lacking a cloth-like appearance. In a gathered state, the nonwoven fabric shows a tendency to return to its original state so that the elastic strands are kept in a slightly stretched state. As a result, stress relaxation occurs. That is, the contractibility of the elastic strands gradually reduces during long-term storage. There is another problem that the elastic strands tend to break or cling to a roller when they are stretched between rollers or transported as wrapped around a roller. Moreover, the elastic strands are relatively thick, giving a rough, uncomfortable feel to the touch.

Apart from the above techniques, an elastically stretchable composite sheet composed of an elastic sheet (an elastically stretchable film or a sheet made of elastically stretchable continuous fibers) and an inelastically extensible fiber aggregate is proposed (see patent document 5). The elastic sheet and the fiber aggregate are bonded at discretely arranged bonds. The constituent fibers of the fiber aggregate are continuous between adjacent bonds. The continuous fibers are independent of each other without being fused together between the bonds. The continuous fibers curve irregularly between adjacent bonds.

Patent document 5 states that, when the elastically stretchable composite sheet is stretched, the stretch of the elastic sheet is not interfered with by the fiber aggregate because the continuous fibers of the fiber aggregate curve irregularly between the bonds. However, since the continuous fibers of the fiber aggregate are independent of one another between the bonds, the elastically stretchable composite sheet has low tensile strength, and the peel strength between the fiber aggregate and the elastic sheet is also low. The continuous fibers are apt to lift between the bonds to make the sheet look fuzzy and unattractive.

     patent document 1 WO00/20206A1
     patent document 2 WO00/20207A1
     patent document 3 WO95/34264A1
     patent document 4 WO01/87214A1
     patent document 5 US 6730390B1

Disclosure of the Invention

The present invention provides a stretch sheet including an extensible nonwoven fabric and a plurality of elastic filaments arranged to extend in one direction without intersecting with each other and bonded to the extensible nonwoven fabric in their substantially nonstretched state over their whole length.

The present invention also provides a process of producing a stretch sheet including the steps of taking off a plurality of molten elastic filaments as spun from spinning nozzles at a predetermined speed while drawing, fusion bonding the elastic filaments to a nonwoven fabric before the filaments solidify such that the elastic filaments extend in one direction in a non-intersection relation to each other to make a composite, and stretching the resulting composite in the extending direction of the elastic filaments to impart stretchability to the composite.

The present invention also provides a process of producing a stretch sheet including the steps of taking off a plurality of molten elastic filaments spun from spinning nozzles at a predetermined speed while drawing, fusion bonding the elastic filaments to a nonwoven fabric before the filaments solidify such that the elastic filaments extend in one direction in a non-intersection relation to each other to make a composite, and stretching the resulting composite in the extending direction of the elastic filaments to impart stretchability to the composite, in which the distance of travel of the spun molten elastic filaments from the tip of the spinning nozzle until contact with the nonwoven fabric is 600 mm or shorter.

Brief Description of the Drawings

[Fig. 1] Fig. 1 is a perspective of an embodiment of the stretch sheet according to the invention, with a part cut away.

[Fig. 2] Fig. 2(a) and Fig. 2(b) are each a cross-section of the stretch sheet of Fig. 1, taken along the stretch direction of the elastic filaments, of which Fig. 2(a) shows a relaxed state, and Fig. 2(b) illustrates a stretched state.

[Fig. 3] Fig. 3 is an SEM image displaying an example of inelastic fibers with the diameters (or cross-sectional areas) varied nearly stepwise.

[Fig. 4] Fig. 4 is a diagram illustrating measurement of melt tension.

[Fig. 5] Fig. 5 schematically illustrates an apparatus preferably used to produce the stretch sheet of Fig. 1.

[Fig. 6] Fig. 6 is a schematic side view of an essential part of the apparatus of Fig. 5.

[Fig. 7] Fig. 7 schematically illustrates a composite being stretched in the apparatus of Fig. 5.

[Fig. 8] Fig. 8 is a schematic showing inelastic fibers being stretched.

[Fig. 9] Fig. 9 illustrates a method of evaluating resistance to sliding of a diaper.

Detailed Description of the Invention

The present invention will be described based on its preferred embodiments with reference to the accompanying drawings. Fig. 1 is a perspective of an embodiment of the stretch sheet according to the invention, with a part cut away.

A stretch sheet 10 of the present embodiment is composed of two nonwoven fabrics- a first nonwoven fabric 11 and a second nonwoven fabric 12- and a number of elastic filaments 13 interposed between the two nonwoven fabrics 11 and 12. Specifically, the stretch sheet 10 has a number of elastic filaments 13 arranged to extend in one direction in non-intersection relation to one another and bonded in a substantially unstretched state to extensible nonwoven fabrics 11 and 12 containing inelastic fibers.

The stretch sheet 10 contains an elastic resin. The elastic resin serves for the stretchability of the stretch sheet 10 and is an essential component of the stretch sheet of the invention. It is preferred that the elastic resin is present in at least the elastic filaments. The elastic resin may be present or absent in the extensible nonwoven fabric. The elastic resin is exemplified by hereinafter described thermoplastic elastomers (e.g., SBS and SIS) and rubber.

The elastic resin content in the elastic filament is preferably 40% by weight or more, more preferably 70% to 100%. In that range, the elastic resin may be modified, for example, to have improved molding properties, to acquire resistance against oxidation, or to be colored, while securing increased bonding strength between the elastic filaments to the nonwoven fabric. The elastic resin content in the extensible nonwoven fabric is preferably 0% to 30% by weight, more preferably 0% to 10% by weight. Incorporating an elastic resin into the nonwoven fabric brings about not only increased strength at retraction but increased bonding strength due to enhanced compatibility to the elastic filament resin. The total content of the elastic resin per unit area i.e., basis weight, in the stretch sheet of the invention is preferably 1 to 25 $g/m^2$, more preferably 4 to 15 $g/m^2$, in terms of stiffness, feel to the touch, lint-freeness in tearing, strength at retraction, bonding strength between the elastic filaments and the nonwoven fabric, and so forth. The total content of the elastic resin per unit area is obtained by the method described later.

The bonding strength between the elastic filaments 13 and the nonwoven fabric 11 is preferably 10 cN/25 mm or more, more preferably 20 to 200 cN/25 mm. The preferred and the more preferred ranges of the bonding strength also apply to the nonwoven fabric 12. When the bonding strength between the elastic filaments 13 to each of the nonwoven fabrics 11 and 12 is 10 cN/25 mm or more, the stretch sheet 10 exhibits secured integrity as a composite so that the elastic filaments 13 are effectively prevented from separating from the nonwoven fabrics 11 and 12 when the stretch sheet 10 is stretched or bonded to another sheet. When the bonding strength between the elastic filaments 13 and the nonwoven fabrics 11 and 12 is more than 200 cN/25 mm, the stretch characteristics of the stretch sheet 10 may be reduced. So, the upper limit of the bonding strength is preferably 200 cN/25 mm. The bonding strength is measured as follows.

Method of measuring elastic filament/nonwoven fabric bonding strength:

(1) When a composite has two nonwoven fabrics:

From a stretch sheet is cut out a specimen measuring 150 mm in the stretch direction and 25 mm in the direction perpendicular to the stretch direction. The specimen is delaminated into two nonwoven fabrics along the stretch direction to about 3 cm from one end. At this time the elastic filaments will integrally accompany whichever nonwoven fabric to which they are bonded more strongly. The ends of the delaminated nonwoven fabrics are held by jaws (initial spacing: 25 mm) of a tensile tester (Autograph AG-1kNIS from Shimadz Corp.) and pulled apart at a speed of 300 mm/min up to a jaw separation of 160 mm. The peel strength is read between jaw separations of 35 mm and 150 mm. In the case when the readings largely fluctuate to have a maximum and a minimum within the measuring section, an average peel strength is calculated from the highest five readings. In the case when no maximum nor minimum appears, an average peel strength is calculated. In a preferred mode of measurement, Autograph AG-1KNIS (from Shimadz Corp.) and Trapezium 2 (software for material testers, from Shimadz Corp.) are used to calculate an average peel strength from the highest five values. According to this preferred method, an average peel strength can be calculated whether or not the data show a maximum and a minimum. Measurement was taken in quintuplicate, and the average is taken as the bonding strength between the elastic filaments and the nonwoven fabrics.

(2) When a composite has a single nonwoven fabric:

Measurement was carried out in the same manner as described in (1) above, except that the nonwoven fabric is held by one of the jaws, and the elastic filaments are held by the other jaw.

Besides having the recited range of bonding strength between the elastic filaments 13 and the nonwoven fabrics 11 and 12, it is preferred for the stretch sheet 10 of the present embodiment to have a strength at 25% retraction, per unit content of the elastic resin, of 1.0cN/ {50mm·(g/m$^2$)} or more, more preferably 2.0 to 10 cN/ {50 mm·(g/m$^2$)}. As used herein, the term "strength at 25% retraction", which will hereinafter be referred to as a 25% retraction strength, is defined to be the strength of the stretch sheet 10 measured when it is retracted to 25% elongation after 50% elongation in one direction (the extending direction of the elastic filaments 13). With the strength at 25% retraction per unit elastic resin content falling within the recited range, when the stretch sheet 10 is used, for example, as an outer cover of a disposable diaper, it is stretched by small force in putting a diaper on a wearer, allowing for easy diaper change, and it exerts a moderate retraction stress, providing a good fit while being worn. The 25% retraction strength per unit elastic resin content in the stretch sheet 10 is measured as follows.

Method of measuring 25% retraction strength per unit elastic resin content of stretch sheet:

A 25% retraction strength of a stretch sheet is obtained by the method described *infra*. Dividing the 25% retraction strength by the content (i.e., weight) of the elastic resin per unit area of the sheet to give a 25% retraction strength per unit elastic resin content of the stretch sheet. The elastic resin content per unit area in the stretch sheet is obtained by dividing the total amount of the elastic resin used to produce the stretch sheet by the area of the stretch sheet. In the case where the elastic resin is extractable from the stretch sheet using, for example, a solvent, the elastic resin content per unit area may be obtained as follows. The elastic resin is extracted from the stretch sheet with, e.g., a solvent, and the composition of the extract is determined by, e.g., NMR analysis. From the analysis results is calculated the content of the elastic resin, which is divided by the area of the stretch sheet. According to this method, not only the elastic resin present in the elastic filaments of the stretch sheet but the elastic resin, if any, in the extensible nonwoven fabrics of the stretch sheet, so that the total amount of the elastic resin present in the stretch sheet may be determined precisely.

A stretch sheet having a bonding strength of 10 cN/25 mm or more between the elastic filaments 13 and the nonwoven fabrics 11 and 12 and a 25% retraction strength per unit elastic resin content of 1.0 cN/ {50 mm·(g/m$^2$)} or more is produced by the process of production described *infra*. To obtain a stretch sheet having the above described interlaminar bonding strength and stretch characteristics in a good balance, what is of particular importance is that the molten elastic filaments should be fusion bonded to the nonwoven fabrics before they solidify. Fusion bonding the elastic filaments 13 to the nonwoven fabrics 11 and 12 largely contributes to the development of the above recited bonding strength and 25% retraction strength. In contrast, a stretch sheet having elastic filaments and nonwoven fabrics unified by a bonding means other than fusion bonding, for example, applying heat and pressure to a stack of nonwoven fabrics and solidified elastic filaments encounters difficulty in achieving the above recited ranges of both the bonding strength and the 25% retraction strength on account of insufficient bite of the fibers constituting the nonwoven fabrics into the elastic filaments.

A stretch sheet 10 having the above recited ranges of the bonding strength between the elastic filaments 13 and the nonwoven fabrics 11 and 12 and the 25% retraction strength per unit elastic resin content may be produced by, for example, using any of the following manipulations (1) to (3) in carrying out the process of production hereinafter described. (1) The nip strength applied in bonding elastic filaments and nonwoven fabrics is controlled. (2) The clearance between a pair of rollers used to bond elastic filaments and nonwoven fabrics is controlled. (3) The distance of travel of a molten elastic filament spun from the tip of a spinning nozzle to the nip (the point of joining with the nonwoven fabrics) is controlled. Any of the manipulations (1) to (3) allows for bonding the elastic filaments to the nonwoven fabrics while keeping the elastic filaments in shape to some extent. In general, fine filaments as in the present invention are more susceptible to damage than film far broader than filaments when joined to nonwoven fabric. As used herein the term "damage" means reduction in cross-sectional area of the elastic filament cut across the stretch direction of the resulting stretch sheet. Such a damage to the elastic filaments leads to a reduction in retraction strength of the stretch sheet. The above mentioned manipulations make it feasible to achieve the recited ranges of bonding strength and 25% retraction strength without inviting such disadvantage.

Besides having the recited ranges of bonding strength between the elastic filaments 13 and the nonwoven fabrics 11 and 12 and of 25% retraction strength per unit elastic resin content, it is preferred for the stretch sheet 10 to have the elastic filaments 13 bonded to the fibers of the extensible nonwoven fabrics 11 and 12 (hereinafter sometimes referred to as nonwoven fibers) at an average bonding ratio of 10% to 60%, more preferably 20% to 50%. A stretch sheet having the average bonding ratio falling within that range exhibits sufficient peel strength and yet maintains a soft feel with a minimum increase in stiffness due to the bonding between the elastic filaments and the fibers of the nonwovens. Furthermore, with the average bonding ratio falling in the recited range, the stretch sheet has stretch characteristics approaching those of the elastic filaments *per se*. Whereas a general stretch sheet containing an elastic resin film has an elastic resin content per unit area of 30 to 80 $g/m^2$ (the 25% retraction strength per unit elastic resin content of the stretch sheet 10 is 0.6 cN/ {50 mm·$(g/m^2)$}), the stretch sheet of the invention achieves a high 25% retraction strength and a high bonding strength with a smaller amount of the elastic resin per unit area.

Techniques for obtaining the recited range of the average bonding ratio between the elastic filaments and the nonwoven fibers include the described manipulations (1) to (3) and, in addition, manipulation (4) : to set the melting temperature (molding temperature) of an elastic resin at 125° to 180°C higher than the melting point of the lowest-melting component of the components constituting the nonwoven fibers, and manipulation (5) : to use a nonwoven fabric having a fiber density of 0.05 to 0.12 $g/cm^3$, which is relatively lower than that of ordinary nonwovens. By lowering the fiber density of the fibers making up the nonwoven fabrics as in manipulation (5), there is obtained a stretch sheet having high strength in retraction in which the elastic filaments are fusion bonded to the fibers of the surface of the nonwovens without causing interference with extension and retraction. The average bonding ratio between the elastic filaments and the nonwoven fibers is determined as follows.

Method of determining average bonding ratio between elastic filaments and nonwoven fibers:

A cross-section of a single elastic filament and surrounding fibers is taken across the stretch direction of a stretch sheet, and the interface between the perimeter of the filament and the fibers is observed under an SEM at a magnification of 100 to 1000 times. The total length of bonded regions between the filament and the fibers along the circumference of the filament and the total length of non-bonded regions along the circumference are measured, from which the bonding ratio of the elastic filament is calculated according to formula:

Bonding ratio (%) of single elastic filament = [total length of bonded regions/(total

length of bonded regions + total length of non-bonded regions)] x 100

The measurement was repeated 10 times for each sample (n=10) to obtain an average bonding ratio between the elastic filaments and the fibers of the nonwoven fabrics.

Back to Fig. 1, the stretch sheet 10 of the present embodiment is composed of two nonwoven fabrics- a first nonwoven fabric 11 and a second nonwoven fabric 12- and a number of elastic filaments 13 interposed between the two nonwoven fabrics. Every filament 13 is bonded to both the first and second nonwoven fabrics 11 and 12. The first nonwoven fabric 11 and the second nonwoven fabric 12 may be the same or different. As referred to above, when the two nonwoven fabrics are said to be "the same", this means that they are totally equal in every attribute including production process, material, thickness and length of constituent fibers, thickness, and basis weight. Two nonwoven fabrics different in any one of these attributes are regarded to be different. The term "elastic" as used herein is intended to mean the ability of an extensible material to retract to a length of 125% or less of its original length when extended to 100% elongation (to double its original length) and released from force.

The nonwoven fabrics 11 and 12 are both extensible. They are configured to be extensible in the same direction as the stretch direction of the elastic filaments 13. The term "extensible" as used herein with respect to nonwoven fabrics is contemplated to include not only a nonwoven fabric whose constituent fibers *per se* are extensible but also a nonwoven fabric whose constituent fibers are not *per se* extensible but which shows extensibility as a whole as a result of debonding of constituent fibers that have been bonded at their intersections, structural change of a three-dimensional structure formed of a plurality of fibers bonded to one another, breaks of the constituent fibers, or straightening out of slack fibers.

The nonwoven fabrics 11 and 12 in web form may previously be made extensible before being joined with the elastic filaments 13. Alternatively, the nonwoven fabrics 11 and 12 in web form may be inextensible before being joined with the elastic filaments 13 and, after being joined with the elastic filaments 13, be subjected to processing to be made extensible. Methods for making a nonwoven fabric extensible include heat treatment, stretching between spaced pairs of rollers, stretching by a bite between corrugating members, and stretching by a tenter. In the light of the hereinafter described preferred process of making the stretch sheet 10, it is preferred that the nonwoven fabrics 11 and 12 are not extensible in the form of webs so as to secure stable transportation of the webs when the elastic filaments 13 are fusion bonded thereto.

Each of the nonwoven fabrics 11 and 12 is extensible, contains substantially inelastic fibers, and is substantially inelastic.

Each elastic filaments 13 is substantially continuous over the whole length of the stretch sheet 10. The elastic filaments 13 contain an elastic resin. The elastic filaments 13 are arranged to extend in one direction in non-intersection relationship to each other. It is acceptable that the elastic filaments 13 unintentionally intersect with each other due to unavoidable fluctuation of conditions in the production of the stretch sheet 10. The individual elastic filaments 13 may extend straight or in a serpentine fashion as long as they do not intersect with one another. The elastic filaments' not intersecting with each other means that there are few intersections. If intersection occurs, there will be a plurality of fibers between the intersections. Usually, it is industrially rare that the fibers present between adjacent intersections have the same length. When the intersecting elastic filaments are stretched, only the shorter of the fibers present between the intersections will be subjected to the stress. It follows that a considerable number of fibers remain non-stretched even though there are many fibers. With the weight being equal, a stretch sheet having a greater number of intersections of the elastic filaments is less retractable. This results in waste of cost. Considering only longitudinal stretch, if there are transversely extending fibers like a net, not only will the transversely extending fibers be useless, but the above described intersections will occur, which will lead to waste of longitudinally extending fibers. The extending direction of the elastic filaments 13 may be coincident with, or perpendicular to, the moving direction of the first and second nonwoven fabrics 11 and 12 in their manufacture. When the stretch sheet 10 is produced in accordance with the preferred process described later, the extending direction of the elastic filaments 13 coincides with the moving direction of the first and second nonwoven fabrics 11 and 12 in their manufacture.

The fact that the elastic filaments 13 are bonded to the nonwoven fabrics 11 and 12, which are extensible, in their substantially unstretched state offers the following advantages. The stretch sheet 10 of the invention is not subject to stress relaxation due to stretch (creeping) and therefore less likely to reduce in stretchability. Secondly, assuming that the elastic filaments 13 bonded to the nonwoven fabrics 11 and 12 in their 100% stretched state (twice its original length) retract to 1.3 times the original length, they are able to re-stretch to only 1.54 times the original length in their sandwiched state. In contrast, when they are bonded in their non-stretched state, they are able to stretch to the maximum extension length of the nonwoven fabrics 11 and 12 or to the maximum elongation of the elastic filaments 13 because of the difference in the starting length of the filaments 13 before the stretch sheet is re-stretched.

Bonding the elastic filaments 13 to the nonwoven fabrics 11 and 12 in their unstretched state further offers the following advantage. The stretch sheet 10 of the present embodiment may be produced by, for example, bonding elastic filaments 13 in their substantially unstretched state to non-elongated nonwoven fabrics 11 and 12 to form a precursor web, taking up the web in roll (in this stage, the non-elongated nonwoven fabrics 11 and 12 having the elastic filaments 13 bonded therebetween are still non-stretchable), subjecting the precursor web to stretch processing (e.g., corrugation) in a separate step to render the non-elongated nonwoven fabrics 11 and 12 extensible. The precursor web in roll form is not subject to relaxation due to stretch during long-term storage because the precursor web is non-elongated and non-stretchable so that no outer force is exerted to the elastic filaments 13 during storage.

The elastic filaments 13 can be synthetic or natural rubber threads. They may be dry-spun (melt spun) or wet-spun threads. In the light of the preferred process described later, the elastic filaments 13 are preferably those as obtained by melt spinning without being wound up or stored.

The elastic filaments 13 are preferably those formed by drawing a filamentous molten resin as spun from nozzles. On being drawn, the filament has its high-molecular-weight molecules oriented along the longitudinal direction and comes to have an increased extension/retraction ratio of strength at 50% elongation, namely, a decreased hysteresis loss. Drawing also produces fine filaments. For these considerations, the elastic filaments 13 are preferably obtained by drawing the spun filaments 1.1 to 400 times, more preferably 4 to 100 times. In contrast, patent document 3 employs undrawn elastic strands as extruded from the die to be bonded to sheeting so that the hysteresis loss of the elastic strands is not sufficiently small. Patent document 4 has a problem that the sticky resin being drawn comes into direct contact with a roller, around which the strand can wind itself.

It is particularly preferred that the elastic filaments 13 are those formed by drawing a filamentous elastic resin in a molten or softened state. Drawing an elastic resin in a molten or softened state makes it possible to sufficiently reduce the diameter of the filaments and to provide the stretch sheet with a good feel for the reason described later. Furthermore, the thus drawn elastic filaments 13 do not exert contracting force after they are fixed to the nonwoven fabrics 11 and 12 and cooled to ambient temperature. This is equivalent to bonding the elastic filaments 13 to the nonwoven fabrics 11 and 12 in their non-stretched state. In the practice of the present embodiment, drawing operation is carried out, for example, by (a) melt spinning a resin providing elastic filaments 13 into undrawn filaments and re-heating the undrawn filaments to or above the softening point (glass transition point Tg of the hard segments), at which they are drawn or (b) melt spinning a resin providing elastic filaments 13 and directly drawing the resulting molten resin streams. When the stretch sheet 10 is produced by the preferred process described *infra*, the elastic filaments 13 are obtained by directly drawing molten resin filaments as obtained by melt spinning.

The elastic filament 13 as obtained by spinning followed by drawing preferably has a diameter of 10 to 200 µm, more preferably 20 to 130 µm, taking into consideration the feel of the stretch sheet 10 and productivity of the elastic filaments 13. Too thick elastic filaments 13 may cause a perceptible level difference in the feel to the touch on the surface of the stretch sheet 10. Such a level difference adversely affects the feel of the stretch sheet 10. From this point of view, the smaller the diameter of the elastic filaments 13, the more perceptible the feel of only the nonwoven fabrics 11 and 12. To use finer elastic filaments 13 is also favorable in terms of reducing light transmittance of the nonwoven fabrics thereby to provide the nonwoven fabrics with properties of hiding the color of a body fluid. For the elastic filaments 13 to have a smaller diameter is also favorable in terms of preventing them from damages (cracking or breaking) when stretched between corrugating rollers for elasticity development. More specifically, the diameter of the filaments 13 is preferably equal to or less than the clearance between the ridges of the corrugating rollers. In order to achieve a high stretch ratio by the depth of engagement while securing durability of the corrugating rollers, the clearance is preferably as small as 250 µm or less, more preferably 200 µm or less. The ratio of the diameter of the elastic filament to the clearance is preferably 0.2 to 1, more preferably 0.2 to 0.5. Nevertheless, finer elastic filaments 13 are more difficult to produce. For all these considerations, it is preferred that the diameter of the elastic filaments 13 falls within the above recited range.

To prevent the above described level difference, the ratio of the diameter of the elastic filaments 13 as measured in the thickness direction of the stretch sheet to the thickness of the stretch sheet 10 is preferably 1% to 30%, more preferably 5% to 12%.

The elastic filaments 13 may have a circular cross-section or, as the case may be, an oval or a flattened cross-section. When the stretch sheet 10 is produced by the process hereinafter described, the elastic filaments 13 tend to have a flat cross-sectional shape. In such a case, it is preferred that the elastic filament 13 is shaped such that the major axis of the flat cross-section is nearly oriented in a planar direction of the stretch sheet 10 and the minor axis is nearly along the thickness direction of the stretch sheet 10.

In the case where the elastic filaments 13 have a flat cross-section, the major to minor axis ratio (average flatness) is preferably 1.0 to 7.0, more preferably 1.1 to 3.0, in view of stretch characteristics, bonding strength between the elastic filaments 13 and the nonwoven fabrics 11 and 12, and hiding properties of the stretch sheet 10. The elastic filaments 13 having a flattened cross-section are arranged with the major axis of their cross-section generally coinciding with a planar direction of the stretch sheet 10. With respect to the elastic filament having a flat cross-section, the term "diameter" means an average of the longer axis and the minor axis. The term "major axis" as used with respect to a flat cross-section of an elastic filament 13 designates the dimension of the longest transversal of the cross-section as observed under a microscope. The term "minor axis" means the dimension of the short sides of a circumscribed rectangle circumscribing the cross-section and having two sides parallel to the longest transversal. Arbitrarily chosen five points of elastic filaments were measured for major and minor axes to calculate their flatnesses, from which an average flatness is calculated. The average of the measured diameters is taken as the diameter of the elastic filaments.

In one of the preferred embodiments, the elastic filaments 13 may be colored differently from the first and second nonwoven fabrics 11 and 12. In this case, the elastic filaments 13 will be seen through the first nonwoven fabric 11 and/or the second nonwoven fabric 2 and look like a stripe pattern to provide a decorative design. This decorative effect is particularly appreciable when the thickness and basis weight of the nonwoven fabrics fall in the ranges described *infra*.

To provide the stretch sheet 10 with sufficient stretch characteristics and a good cloth-like feel and, if desired, the above mentioned decorative design, it is preferred that the elastic filaments 13 are arranged at a pitch (a distance between adjacent elastic members 13) of 0.1 to 5 mm, more preferably 0.4 to 1 mm, provided that their diameter is in the above recited range.

The elastic filaments 13 are bonded to the nonwoven fabrics 11 and 12 over their whole length. That is, the bonds between each elastic filament 13 and the nonwoven fabrics 11 and 12 are continuous over the whole length of the elastic member 13. As used herein, the expression "over the whole length" is not intended to mean that all the fibers in contact with an elastic filament 13, of the fibers constituting the nonwoven fabrics 11 and 12, should be bonded to the elastic filament 13 but is intended to mean that the elastic filament 13 and the surrounding fibers of the nonwoven fabrics 11 and 12 are bonded to each other without intentionally leaving part of them unbonded. Bonding the elastic filament to the nonwoven fabrics 11 and 12 over its whole length achieves sufficiently ensured adhesion between the elastic filament and each of the nonwoven fabrics 11 and 12. Thus the elastic filaments 13 are prevented from separating from the nonwoven fabrics 11 and 12 when the stretch sheet 10 is stretched. If the elastic filaments 13 separate from the nonwoven fabrics 11 and 12, the nonwoven fabric may lift to cause the stretch sheet 10 to wrinkle and lose integrity.

Modes of bonding the elastic filament 13 and the first and second nonwoven fabrics 11 and 12 include fusion bonding. In the preferred process of producing the stretch sheet 10 hereinafter described, bonding the elastic filaments 13 and the first and second nonwoven fabrics 11 and 12 is carried out by fusion bonding. The state obtained by "fusion bonding" as contemplated herein is a state in which both the elastic filament and the fiber of the nonwoven fabrics 11 and 12 are fused and thereby bonded together, a state in which either one of the elastic filament and the fiber is fused to let the other bites therein to achieve bonding, or a mixed state thereof. According to this mode of bonding, application of excess heat to the nonwoven fabrics 11 and 12 is avoided, and since the elastic filaments 13 as melt-spun are fusion bonded to the nonwoven fabrics before they solidify, only the fibers present around each elastic filament 13 are fusion bonded to the elastic filament 13, whereas more remote fibers are not. Thus, the good feel essentially possessed by the nonwoven fabrics is retained to provide the resulting stretch sheet 10 with a good feel. In this case, an adhesive may be applied as an auxiliary bonding means before the nonwoven fabrics 11 and 12 and the elastic filaments 13 are bonded. After the nonwoven fabrics 11 and 12 and the elastic filaments 13 are bonded, the resulting laminate may be subjected to heat treatment (e.g., steam jet treatment or heat embossing) or mechanical entanglement (e.g., needle punching or hydroentanglement) as an auxiliary bonding means. These auxiliary bonding means can impair the feel of the resulting stretch sheet 10 or damage the elastic filaments 13. It is therefore preferred that the elastic filaments 13 are fusion bonded to the nonwoven fabrics 11 and 12 only by the heat of fusion of the elastic filaments 13. However, heat treatment by blowing hot air in a through-air system is a preferred auxiliary bonding means, which does not impair the feel of the stretch sheet 10 and ensures the bonding strength of the nonwoven fabrics 11 and 12.

The stretch sheet 10 is stretchable in the same direction as the extending direction of the elastic filaments 13. The stretch of the stretch sheet 10 owes to the elasticity of the elastic filaments 13. When tension is applied to the stretch sheet 10 in the same direction as the extending direction of the elastic filaments 13, the elastic filaments 13 and the first and second nonwoven fabrics 11 and 12 stretch. On removal of the tension, the elastic filaments retract, and with it the first and second nonwoven fabrics 11 and 12 return to their original unstretched configuration.

Unlike the sheets disclosed in patent documents 1 and 2, the stretch sheet 10 of the present embodiment has no other elastic filaments bonded in a perpendicular relation with respect to the elastic filament 13. Therefore, when stretched in the same direction as the extending direction of the elastic filaments 13, it stretches without substantial width contraction. In other words, the stretch sheet 10, while being stretched, has an almost equal width over the whole length thereof. As a result, the stretch sheet 10 has ease of handling when it is transported and processed in its stretched state. When the stretch sheet 10 is used as, for example, an outer cover of a pull-on diaper, the diaper is effectively prevented from sliding down or wrinkling while being worn. Considering the structure of a diaper and the motion of a wearer, a stretchable diaper experiences non-uniform stretch around the circumferential direction, but the diaper using the stretch sheet 10 undergoes little contraction in width, whereby sliding down or wrinkling of the diaper is prevented effectively. From this point of view, when the stretch sheet 10 is stretched, for example, 1.5 times the original length, the width contraction of the stretch sheet 10 is preferably 1% to 10%, more preferably 1% to 5%, based on the width before stretch. The width contraction is calculated using formula: width contraction (%) = (1- width after stretch/width before stretch) x 100. The width after stretch is measured as follows. A sample measuring longer than 150 mm generally in the machine direction (within an angle of $\pm 15°$ with respect to the machine direction) and 50 mm in width is cut out. The sample is held at its longitudinal ends by jaws with an initial jaw separation of 150 mm while maintaining its width at 50 mm, taking care so that the sample may neither sag nor stretch in its longitudinal direction. The sample in this state is stretched 1.5 times the initial jaw separation, and the width is measured at the middle of the stretched sample.

Figs. 2(a) and 2(b) each represent a cross-section of one embodiment of the stretch sheet 10 of the present invention, taken along the extending direction of the elastic filaments 13. The form illustrated in Figs. 2(a) and 2(b) is the one which appreciably forms when corrugating members are used to carry out processing for elasticity development, one of the processing steps for the production of the stretch sheet 10. Fig. 2(a) is a cross-section of the stretch sheet 10 in its natural (relaxed) state, and Fig. 2(b) in its stretched state. In the relaxed state, the stretch sheet 10 takes on a wavy form made of alternating crests 14' and valleys 14". Each crest 14' connects with each groove 14" via a ridgeline 15'. The stretch sheet 10 is slightly thinner and therefore more light-transmissive at the ridgelines than at the crests 14' and the valleys 14". In a plan view of the stretch sheet 10, the crests 14', ridgelines 15', and valleys 14" extend in the direction perpendicular to the stretch direction of the stretch sheet 10. As a result, a dim stripe pattern appears on the stretch sheet 10 in its relaxed state, the stripe being composed of more light-transmissive ridgelines 15' and less light-transmissive crests 14' and valleys 14". In some cases depending on the conditions, e.g., of processing for elasticity development, the stripe pattern may become more appreciable on stretching the stretch sheet 10 as discussed below.

In Fig. 2(b), the stretch sheet 10 in a stretched state is composed of high basis weight portions 14 and low basis weight portions 15 alternating each other in the extending direction of the elastic filaments 13. The high basis weight portions 14 extend in stripes in the direction perpendicular to the extending direction of the elastic filaments 13, and so do the low basis weight portions 15. The high basis weight portions 14 alternate with the low basis weight portions 15 at a given frequency. The high basis weight portions 14 have those protruding upward and those protruding downward alternating each other. The high basis weight portions 14 protruding upward correspond to the crests 14' of the sheet 10 in the relaxed state shown in Fig. 2(a). The high basis weight portions 14 protruding downward correspond to the valleys 14" of the sheet 10 in the relaxed state shown in Fig. 2(a). The low basis weight portions 15 correspond to the ridgelines 15' of the sheet 10 in the relaxed state shown in Fig. 2(a). The high basis weight portions 14 and the low basis weight portions 15 have different degrees of light transmission based on the difference in basis weight, thereby assuming a stripe pattern extending in the direction perpendicular to the extending direction of the elastic filaments 13, providing an improved design. Since the stretch sheet 10 assumes a stripe pattern based on the elastic filaments 13 as stated previously, the stretch sheet 10 exhibits a grid pattern composed of this stripe pattern and the stripe pattern based on the high basis weight portions 14 and the low basis weight portions 15, providing a further improved design. In a preferred embodiment of the invention the regions protruding upward or downward are filled with fibers as illustrated in Figs. 2 (a) and 2(b).

The high basis weight portions 14 have a higher basis weight and a larger thickness than the low basis weight portions 15. As a result, the high basis weight portions 14 and the low basis weight portions 15 have different levels of light transmission, assuming a stripe pattern. The high basis weight portions 14 have a substantially equal width; so do the low basis weight portions 15.

The thickness of the high basis weight portions 14 is preferably 0.3 to 10 mm, more preferably 0.5 to 1 mm. The thickness of the low basis weight portions 15 is preferably 0.1 to 3 mm, more preferably 0.2 to 0.6 mm, in terms of stretch characteristics and breathability. The thickness measurement is taken as follows. The stretch sheet 10 having been left in an environment of $20°\pm2°C$ and $65\%\pm5\%$ RH for at least two days is stretched 1.5 times in its stretch direction and sandwiched between flat plates under a load of $0.5$ cN/cm$^2$ in the stretched state. Three fields of cross-sections of the sheet are observed under a microscope at a magnification of 50 to 200 times to obtain an average thickness for each of the fields, and an average of the values of the three fields is then obtained. The high basis weight portions 14 and the low basis weight portions 15 easily form when the stretch sheet 10 is produced in accordance with the process hereinafter described.

Materials of the first and second nonwoven fabrics 11 and 12 and the elastic filaments 13 that compose the stretch sheet 10 will then be described. The fibers that can be used to make the nonwoven fabrics 11 and 12 are substantially inelastic fibers, including fibers of polyethylene (PE), polypropylene (PP), polyesters (PET and PBT), and polyamides. The fibers composing the nonwoven fabrics 11 and 12 may be staple fibers or continuous fibers and hydrophilic or water repellent. Sheath/core or side-by-side conjugate fibers, split fibers, modified cross-section fibers, crimped fibers, and heat shrunken fibers are also useful. These fibers may be used either individually or in combination of two or more thereof. The nonwoven fabrics 11 and 12 may be nonwoven fabrics of continuous filaments or staple fibers. The nonwoven fabrics 11 and 12 are preferably formed of staple fiber nonwoven fabrics to provide a thick and bulky stretch sheet 10. In applications where the stretch sheet 10 is used as a member adapted to be brought into contact with the skin of a wearer, staple fiber nonwoven fabric with good feel may be used on the side adapted to be brought into contact with the skin, whilst continuous filament nonwoven fabric with high strength may be used on the opposite side.

It is preferred that the fibers composing each nonwoven fabric 11 or 12 are made up of at least two components including a low melting component and a high melting component. At least the low melting component is fused to bond the fibers at their intersections. Preferred examples of a sheath/core conjugate fiber made up of at least two components including a low melting component and a high melting component are those having a high melting PET or PP as a core and a low melting PET, PP or PE as a sheath. Using such conjugate fibers is particularly preferred to enhance the fusion bonding to the elastic filaments 13 and reduce likelihood of debonding from the elastic filaments 13.

The thickness of each of the nonwoven fabrics 11 and 12 is preferably 0.05 to 5 mm, more preferably 0.1 to 1.0 mm, even more preferably 0.15 to 0.5 mm. The thickness measurement is taken as follows. The stretch sheet 10 is sandwiched between flat plates under a load of $0.5$ cN/cm$^2$. Three fields of a cross-section of the sheet are observed under a microscope at a magnification of 50 to 200 times to obtain an average thickness for each of the fields, and an average of the values of the three fields is then obtained. The total thickness of the sheet is obtained by measuring the distance between the plates. The basis weight of each nonwoven fabric 11, 12 is preferably 3 to 100 g/m$^2$, more preferably 10 to 30 g/m$^2$, in terms of feel, thickness, and design.

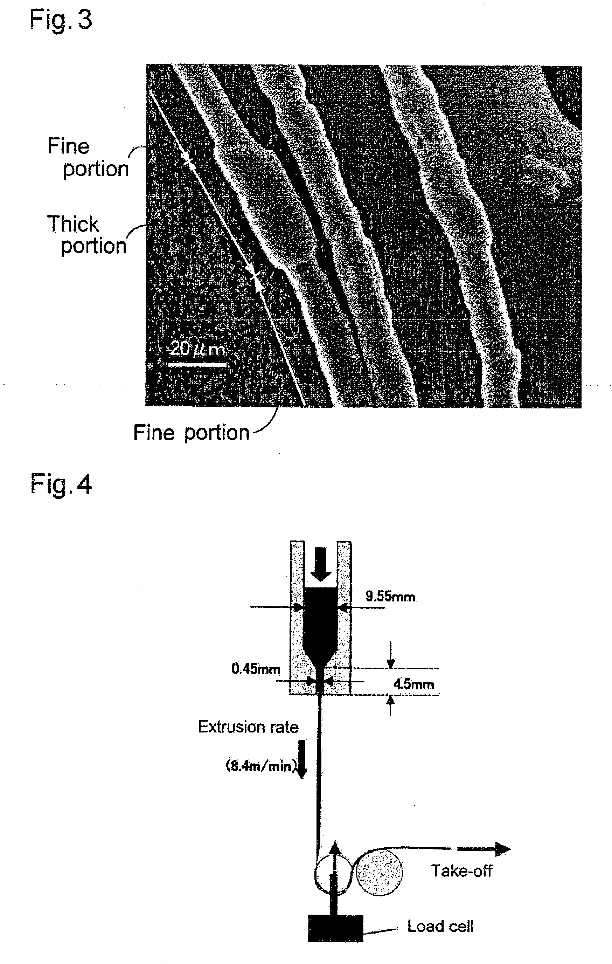

Each nonwoven fabric 11 or 12 is preferably fabricated of substantially inelastic fibers in terms of feel and non-stickiness and the like. The proportion of the inelastic fibers in the nonwoven fabric is preferably 70% or more, more preferably 90% or more, even more preferably 100%, by weight. The substantially inelastic fiber may contain an elastic resin in addition to an inelastic resin. The proportion of the inelastic resin is preferably 70% or more, more preferably 90% or more, even more preferably 100%, by weight. It is particularly preferred to use inelastic fibers having a varied thickness along their length (hereinafter referred to as varied thickness fibers). The individual varied thickness fiber includes portions with a larger cross-sectional area (or diameter) and portions with a smaller cross-sectional area (or diameter) along its length. The varied thickness fiber may have its cross-sectional area (or diameter) continuously varied from the finest portions to the thickest portions, or may have its cross-sectional area (or diameter) varied stepwise like necking observed in drawing undrawn yarn. An example of inelastic fibers having the diameter (cross-sectional area) varied stepwise is shown in Fig. 3.

The inelastic fibers are preferably obtained from fibers having a given diameter and a high elongation (e.g., a maximum elongation of 80% to 800%, preferably 120% to 650%) in view of obtaining the stretch sheet 10 having a high maximum strength. The elongation of a fiber is basically determined in accordance with JIS L-1015 in an environment of $20\pm2°C$ and $65\pm2\%$ RH under conditions of an initial jaw separation of 20 mm and a pulling speed of 20 mm/min. In the case when the fiber to be measured is too short (typically when the fiber to be measured is drawn from a prepared nonwoven fabric) to set the initial jaw separation at 20 mm, i.e., when the fiber is shorter than 20 mm, the jaw separation distance may be set to 10 mm or 5 mm.

The high elongation fibers mentioned above are preferably low-drawn, inelastic fibers. When the stretch sheet 10 of the present embodiment is produced using low-drawn inelastic fibers as precursor fibers in accordance with the process described *infra*, the low-drawn fibers are drawn in processing for developing elasticity to create finer portions and converted to varied thickness fibers. While the processing for elasticity development changes the structure of the nonwoven fabrics into an easily extensible form, the processing also elongates the low-drawn fibers to make the nonwoven fabrics more extensible as a whole. Therefore, destruction of the bonds between the varied thickness fibers and the bonds between the nonwoven fabrics 11 and 12 and the elastic filaments 13 can be minimized thereby to provide the stretch sheet 10 with increased strength while retaining the stretch performance properties. That is, a stretch sheet 10 having both high elongation and high strength can be produced. Additionally, the less likelihood of the bonds between the varied thickness fibers being destroyed during the processing for elasticity development is effective in preventing the nonwoven fabrics 11 and 12 from assuming a fuzzy appearance. This is advantageous for improving the appearance of the stretch sheet 10 of the present embodiment. In contrast to this, the elastically stretchable composite sheet described in patent document 5 (see Background Art) fails to obtain both high elongation and high strength because the solvent weld or mechanical entanglement between the fibers are undone during the step of stretching due to the low drawability of the fibers, resulting in a reduction of sheet strength.

To start with the low-drawn precursor fibers results in a substantial increase of the number (and length) of fine fibers compared with before drawing, whereby the stretch sheet 10 of the present embodiment exhibits increased light shielding properties. For the stretch sheet 10 to have increased light shielding properties is advantageous in that, when used as, for example, a topsheet of an absorbent article, such as a sanitary napkin or a disposable diaper, it is more capable of hiding a body fluid absorbed by an absorbent pad from view.

When the varied thickness fibers have their thickness varied periodically, there is produced an additional advantage that the nonwoven fabrics 11 and 12 have a crepe texture with a pleasant feel. In this case, the interval of the thickness changes, i.e., a distance from a thick portion and an adjacent thick portion is preferably 0.5 to 10 mm, more preferably 2 to 5 mm. The pitch can be measured by microscopic observation of the nonwoven fabrics 11 and 12. The diameters of the varied thickness fiber can be determined in accordance with the procedures (1) to (5) :

(1) A sample measuring at least 5 mm square was cut out of the stretch sheet 10 or of only the nonwoven fabric 11 or 12 from the stretch sheet 10.

(2) The sample is affixed to the sample stage of an SEM. To help easy observation, the sample may be stretched to a degree not to destroy the structure (to a degree to remove any slack) and fixed as such to the stage with a double-sided adhesive tape. The degree of stretch here is lower than about the stretch ratio at which a precursor of the stretch sheet 10 is stretched in a stretch processing in the case when the process of producing the stretch sheet 10 includes processing for elasticity development.

(3) SEM observation is carried out at a magnification of 200 times in 5 fields each having an area of at least about 0.4 mm by 0.4 mm.

(4) Fibers are extracted at random. The diameter of each fiber is measured at 20 points at an interval of 0.1 $\mu$m, except interfiber fusion bonds and destroyed parts. At least 4 fibers are measured per field (a total of 20 fibers in 5 fields).

(5) The maximum and minimum diameters are obtained for each of the twenty fibers. A fiber having a difference of 1 $\mu$m or greater between the maximum and minimum diameters and, when the diameter variation is plotted against the axial direction, shows the maximum or minimum diameter at two or more points is called a varied thickness fiber.

In order to further ensure the above described effects, it is preferred that the varied thickness fibers have a thickness of 2 to 15 $\mu$m, more preferably 5 to 12 $\mu$m, at the finest portion and of 10 to 40 $\mu$m, more preferably 12 to 30 $\mu$m, at the thickest portion. The difference between the maximum diameter and the minimum diameter of the varied thickness fibers is preferably 3 $\mu$m or greater, more preferably 5 $\mu$m or greater, even more preferably 10 $\mu$m or greater. The varied thickness fibers preferably have a diameter ratio, being represented in terms of maximum diameter/minimum diameter, of 1 to 15, more preferably 1.2 to 10, even more preferably 2 to 5.

It is preferred for the varied thickness fibers to have a higher interfiber fusion bonding strength than their strength at 100% elongation so that the fusion bonds between the fibers of the stretch sheet 10 before being processed for elasticity development (stretch sheet precursor) may not be destroyed to reduce the strength of the nonwoven fabrics when the stretch sheet precursor is stretched for elasticity development. The fusion bonding strength can be measured by the method taught in commonly assigned JP 2004-218183A, paragraph. [0040]. The strength at 100% elongation is measured using a tensile tester at an initial jaw separation of 20 mm and a pulling speed of 20 mm/min.

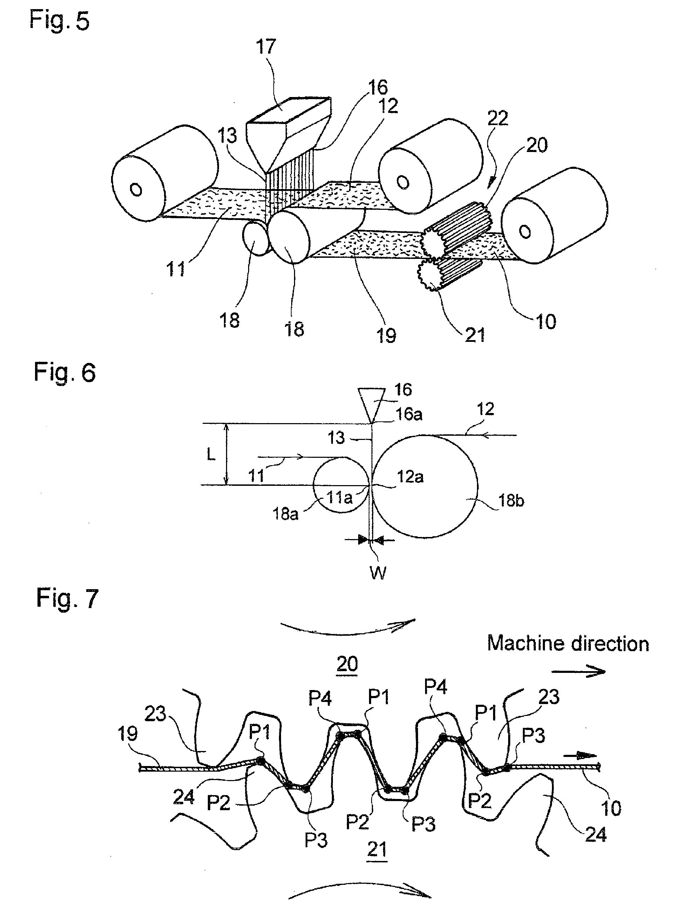

The term "low-drawn inelastic fibers" as referred to above is intended to include both fibers having been drawn at a low draw ratio after spinning and fibers not having been drawn at all, namely undrawn fibers. As previously described, it is preferred to use low-drawn fibers having an elongation of 80% to 800%, more preferably 120% to 650%. Low-drawn fibers the elongation of which is within that range are successfully drawn through a stretching unit 22 shown in Fig. 5 (hereinafter described) into the varied thickness fibers. The diameter of the low-drawn fibers is preferably 10 to 35 $\mu$m, more preferably 12 to 30 $\mu$m.

The varied thickness fibers are, as previously described, preferably obtained from low-drawn fibers with a given fiber diameter. The low-drawn fibers may be single-component fibers or conjugate fibers made of two or more materials, such as sheath/core conjugate fibers or side-by-side conjugate fibers. Conjugate fibers are preferred, taking into consideration ease of bonding the varied thickness fibers one another and ease of bonding between the unwoven fabrics 11 and 12 and the elastic filaments 13. In using sheath/core conjugate fibers, those having a polyester (e.g., PET or PBT) or polypropylene (PP) core and a low melting polyester (e.g., PET or PBT), polypropylene (PP) or polyethylene (PE) sheath are preferred; for they are strongly fusion-bondable to the elastic filaments 13 which contain an polyolefinic elastomer, thereby preventing delamination.

The varied thickness fibers may be staple fibers or continuous fibers. In light of the preferred process of producing the stretch sheet 10 hereinafter described, it is preferred to use staple fibers. The varied thickness fibers may be hydrophilic or water repellent.

The nonwoven fabrics 11 and 12 may be each composed solely of the varied thickness fibers or may further contain other inelastic fibers with a constant thickness. Examples of the other inelastic fibers are as hereinbefore described. Where the nonwoven fabric 11 or 12 contains inelastic fibers with a constant thickness in addition to the varied thickness fibers, the mixing ratio of the other inelastic fibers is preferably 1% to 30%, more preferably 5% to 20%, by weight.

The varied thickness fibers are desirably present in both the nonwoven fabrics 11 and 12 but may be present in only one of them.

The elastic filaments 13 are made from elastic resins including thermoplastic elastomers and rubbers as previously described. The elastic filaments 13 are continuous fibers having elasticity. Thermoplastic elastomers are particularly suitable as an elastic resin. This is because, for one thing, thermoplastic elastomers are melt-spinnable using an extruder in the same manner as ordinary thermoplastic resins. For another, the fibers thus obtained are easy to fusion bond and therefore suited for use in the stretch sheet of the present embodiment. Examples of the thermoplastic elastomers include styrene elastomers, such as SBS (styrene-butadiene-styrene), SIS (styrene-isoprene-styrene), SEBS (styrene-ethylene-butadiene-styrene), and SEPS (styrene-ethylene-propylene-styrene); olefin elastomers, such as ethylene-based $\alpha$-olefin elastomers and propylene-based elastomers comprising ethylene, butene, octene, etc.; polyester elastomers; and polyurethane elastomers. These elastomers may be used either individually or in combination of two or more thereof. Sheath/core or side-by-side conjugate fibers composed of these resins are also useful. Fibers made from a styrene elastomer, an olefin elastomer or a combination thereof are particularly preferred in view of spinnability, stretch characteristics, and cost.

It is preferred to use a combination of elastic filaments 13 of an SEBS resin or an SEPS resin and nonwoven fabrics 11 and 12 of PE sheath/PP core conjugate fibers or PE sheath/PET core conjugate fibers. In the case of using this combination, fusion bonding is ensured, and the conjugate fibers do not melt completely (the core remains non-fused) because of the high melting point of the core, thereby to provide the stretch sheet 10 with a high strength.

It is particularly preferred to use elastic filaments 13 made from a resin composition containing a A1-B-A2 triblock copolymer, in which the polymer blocks A1 and A2 are composed mainly of aromatic vinyl polymer, and the polymer block B is composed mainly of a polyolefin.

The sum of the weight average molecular weight (hereinafter abbreviated as Mw) of the polymer block A1 and that of the polymer block A2 as measured by gel permeation chromatography (GPC) is preferably 14000 to 20000, more preferably 14000 to 18000, and the Mw of the polymer block B as measured by GPC is preferably 40000 to 80000, more preferably 40000 to 70000. The Mw measured by GPC as referred to herein is a polystyrene equivalent molecular weight. GPC is carried out under conditions of: column, TSKgel G-2000H; column temperature, 40°C; mobile phase, tetrahydrofuran (THF); flow rate, 1.0ml/min; sample concentration, 10 mg/5 ml-THF (a sample weighting 10 mg is dissolved in 5 ml of THF at ambient temperature for 10 minutes, followed by filtration through a sintered filter having a pore size of 0.45 $\mu$m); and amount of injection, 500 $\mu$l.

In the case where the resin composition contains two or more kinds of A1-B-A2 triblock copolymers, the "Mw of the polymer block A1 as measured by GPC" is a weight average of the Mws of the polymer blocks A1 of the two or more A1-B-A2 block copolymers. More specifically, when, for instance, the resin composition contains an A1'-B'-A2' triblock copolymer and an A1"-B"-A2" triblock copolymer in proportions of 80% by weight and 20% by weight, respectively, based on the total A1-B-A2 triblock copolymer content, the "Mw of the polymer block A 1 as measured by GPC" is calculated from formula: (Mw of polymer block A1' measured by GPC) x 0.8 + (Mw of polymer block A1" measured by GPC) x 0.2.

Where the resin composition contains two or more kinds of A1-B-A2 triblock copolymers, the "Mw of the polymer block A2 as measured by GPC" and the "Mw of the polymer block B as measured by GPC" are obtained in the same manner as for the polymer block A1.

Where the resin composition contains two or more kinds of the A1-B-A2 triblock copolymers, the "sum of the Mws of the polymer blocks A1 and A2 as measured GPC" is the sum of the thus calculated Mws of the polymer blocks A1 and of A2.

The resin composition containing the A1-B-A2 triblock copolymer satisfying the aforementioned physical properties exhibits good spinnability to form the elastic filaments 13 without breakage even with a small diameter and without vibration after being extruded from spinning nozzles.

The polymer block A1 is composed mainly of an aromatic vinyl polymer. Examples of aromatic vinyl monomers providing the aromatic vinyl polymer include styrene, p-methylstyrene, m-methylstyrene, p-t-butylstyrene, $\alpha$-methylstyrene, chloromethylstyrene, p-t-butoxystyrene, dimethylaminomethylstyrene, dimethylaminoethylstyrene, vinyltoluene, 1-vinylnaphthalene, 4-propylstyrene, 4-cyclohexylstyrene, 4-dodecylstyrene, 2-ethyl-4-benzylstyrene, and 4-(phenylbutyl) styrene. The aromatic vinyl polymer may be composed of one or more than one of these monomers. Styrene is preferred of the recited aromatic vinyl monomers in terms of ease of polymerization and availability.

The content of the aromatic vinyl polymer in the polymer block A1 is preferably 90% to 100%, more preferably 95% to 100%, based on the total repeating units of the polymer block A1.

The polymer block A1 may contain a conjugated diene polymer in addition to the aromatic vinyl polymer. Examples of conjugated diene monomers providing the conjugated diene polymer include 1, 3-butadiene, isoprene, 1, 3-pentadiene, 2, 3-dimethyl-1, 3-butadiene, 2-methyl-1, 3-pentadiene, 1, 3-hexadiene, phenylbutadiene, 4, 5-diethyl-1, 3-octadiene, and 3-butyl-1, 3-octadiene. The conjugated diene polymer may be composed of one or more than one of these conjugated diene monomers.

The content of the conjugated diene polymer in the polymer block A1 is preferably 0% to 10% by weight, more preferably 0% to 5% by weight, based on the total repeating units of the polymer block A1.

The Mw of the polymer block A1 measured by GPC is preferably 7000 to 10000 more preferably 7000 to 9000.

The polymer block A2 may be the same as the polymer block A1. Unless otherwise specified, the description of the polymer block A1 applies to the polymer block A2. The polymer blocks A1 and A2 may be structurally the same or different.

It is particularly preferred that both the polymer blocks A1 and A2 be polystyrene. That is, the elastic filaments 13 preferably comprise only polystyrene as the polymer block A1 and only polystyrene as the polymer block A2.

The total content of the polymer blocks A1 and A2 in the A1-B-A2 triblock copolymer is preferably 15% to 40%, more preferably 18% to 30%, by weight, based on the total repeating units of the A1-B-A2 triblock copolymer.

In the case where both the polymer blocks A 1 and A2 are polystyrene blocks, the block A1 to block A2 weight ratio (A1/A2) in the A1-B-A2 triblock copolymer is preferably 0.8 to 1.2, more preferably 0.9 to 1.1.

The polymer block B is composed mainly of a polyolefin. Examples of olefin monomers providing the polyolefin include isoprene, butadiene, ethylene, propylene, and butylene. The polymer block B may be derived from one or more than one of these olefin monomers. Ethylene and propylene are preferred olefin monomers in terms of weatherability and stretch characteristics.

The content of the polyolefin in the polymer block B is preferably 90% to 100%, more preferably 95% to 100%, by weight based on the total repeating units of the polymer block B.

The polymer block B may contain other polymers polymerizable with the polyolefin and derived from other polymerizable monomers exemplified by styrene. The other polymer polymerizable with the polyolefin constituting the polymer block B may be derived from one or more than one of such other polymerizable monomers.

The content of the other polymer in the polymer block B is preferably 0% to 10%, more preferably 0% to 5%, by weight based on the total repeating units of the polymer block B.

Examples of the polymer block B include polyisoprene, polybutadiene, poly(ethylene-propylene), and poly (ethylene-butylene). Preferred of them is poly(ethylene-propylene) for the weatherability and stretch characteristics.

The content of the polymer block B in the A1-B-A2 triblock copolymer is preferably 60% to 85%, more preferably 70% to 82%, by weight based on the total repeating units of the A1-B-A2 triblock copolymer.

Examples of preferred A1-B-A2 triblock copolymers are a styrene-butadiene-styrene block copolymer (SBS), a styrene-isoprene-styrene block copolymer (SIS), a styrene-ethylene-butylene-styrene block copolymer (SEBS) (a hydrogenation product of SBS), a styrene-ethylene-propylene-styrene block copolymer (SEPS) (a hydrogenation product of SIS), and a styrene-ethylene-ethylene-propylene-styrene block copolymer (SEEPS). Preferred of them are SEBS, SEPS, and SEEPS in view of the excellent heat stability and weatherability of the resulting elastic filaments 13.

The A1-B-A2 triblock copolymer may be a commercially available product. Examples of commercial products that are preferably used as the A1-B-A2 triblock copolymer include Kraton (trade name) from Kraton Polymer, Taftec and Tafprene (trade names) from Asahi Chemical Industry, and Septon (trade name) series and Hybrar (trade name) series, both from Kuraray.

The A1-B-A2 triblock copolymer may be synthesized as follows. An aromatic vinyl compound is put in a hydrocarbon solvent (e.g., cyclohexane) and subjected to anionic polymerization in the presence of an initiator (e.g., an organic lithium compound or metallic sodium) to obtain a polymer block A1. An olefin monomer is added thereto to be polymerized to obtain a polymer block A1-B. In using a conjugated diene as an olefin monomer, hydrogen is added to the double bond of the conjugated diene to obtain a polymer block A1-B as purposed. The degree of hydrogenation of the conjugated dienic double bonds is preferably 80% or more, more preferably 90% or more, in terms of heat resistance and weatherability. The hydrogenation reaction may be carried out using a noble metal catalyst (e.g., platinum or palladium), an organonickel compound, an organocobalt compound, or a complex catalyst composed of such an organometallic compound and other organometallic compound. The degree of hydrogenation is calculated by the test method for iodine value. An aromatic vinyl compound is then added to be polymerized to obtain an A1-B-A2 block copolymer.

The resin composition making the elastic filaments 13 may comprise the above described A1-B-A2 triblock copolymer alone or, if needed, may contain other resin(s) as long as the object of the invention is not damaged. Where the resin composition contains the A1-B-A2 triblock copolymer and other resin(s), the content of the A1-B-A2 triblock copolymer in the resin composition is preferably 90% to 100%, more preferably 95% to 100%, by weight. From the standpoint of obtaining the highest possible spinnability and stretch characteristics, the resin composition preferably comprises the A1-B-A2 triblock copolymer alone.

The resin composition may contain only one kind of the A1-B-A2 triblock copolymer or two or more structurally different kinds of the A1-B-A2 triblock copolymers.

In the case where the resin composition contains other resin(s) in addition to the A1-B-A2 triblock copolymer, the other resins include rubber and thermoplastic elastomers, such as polyolefin elastomers, polyester elastomers, and polyurethane elastomers. Resins other than thermoplastic elastomers, such as thermoplastic resins, e.g., polyethylene and polypropylene, may be usable as well. These other resins may be used either individually or as a combination of two or more thereof.

The resin composition is prepared by melting the A1-B-A2 triblock copolymer or melting a mixture of the A1-B-A2 triblock copolymer and other resins described in various known manners. For instance, the components are put and blended in a Henschel mixer, a V-blender, a tumbler mixer, or a ribbon blender either all at once or successively, and the blend is melt kneaded in a kneading machine, such as a single screw extruder, a multi-screw extruder, a kneader, or a Banbury mixer.

Where necessary, the resin composition may further contain a plasticizer (e.g., a paraffinic oil, a paraffinic wax, a naphthenic oil, an ethylene-$\alpha$-olefin oligomer, or a low molecular polyethylene), a weather stabilizers, a heat stabilizer, an antistatic agent, a lubricant, a slip agent, a nucleating agent, a flame retardant, a pigment, a dye, an organic or inorganic filler, and so forth as long as the object of the invention is not damaged.

When the resin composition in a molten state at 245°C is extruded from an orifice of a cylindrical nozzle of 0.45 mm in inner diameter and 4.5 mm in length at a rate of 8.4 m/min, the maximum melt tension of the resin composition is preferably 0.06 to 0.20 cN, more preferably 0.07 to 0.15 cN. A method of measuring a melt tension is illustrated in Fig. 4. The upward force applied to the load cell shown in Fig. 4 is measured as a melt tension. With the maximum melt tension of the resin composition making the elastic filaments 13 being in the above recited range, the resin composition exhibits better spinnability. The melt tension is adjusted by addition of, e.g., a plasticizer (e.g., paraffin), rubber, or a thermoplastic resin. The resin composition having the above described composition has a melt tension falling within the recited range.

The apparent basis weight of the elastic filaments formed of a number of the elastic filaments 13 interposed between the two nonwoven fabrics 11 and 12 is preferably 4 to 30 g/m$^2$, more preferably 6 to 15 g/m$^2$, in terms of stretch characteristics, feel, thickness, and cost.

A preferred process for producing the stretch sheet 10 of the present embodiment is then described with reference to Fig. 5. In this process, a number of molten elastic filaments 13 spun from spinning nozzles 16 are fusion bonded to nonwoven fabrics 11 and 12 before solidifying while being drawn by taking off at a predetermined speed as oriented in one direction in a non-intersection relation to each other to make a composite 19 of the nonwoven fabrics 11 and 12 with the elastic filaments 13 fusion bonded therebetween. The composite 19 is then subjected to processing for elasticity development in the extending direction of the elastic filaments 13 to impart stretchability to the composite 19.

The spinning nozzles 16 are fitted to a spinning head 17. The spinning head 17 is connected to an extruder. A resin may be supplied to the spinning head 17 via a gear pump. The elastic resin is melt kneaded in the extruder and fed to the spinning head 17. A number of the spinning nozzles 16 are fitted into the spinning head 17 in a straight linear arrangement along the transverse direction of webs of the first and second nonwoven fabrics 11 and 12. The spacing between adjacent spinning nozzles 16 corresponds to the spacing between adjacent elastic filaments 13 in the stretch sheet 10. The orifice of each spinning nozzle 16 is usually circular. The diameter (inner diameter) of the orifice influences the diameter and draw ratio of the elastic filament 13. From this viewpoint, the spinning nozzle 16 preferably has an orifice inner diameter of 0.1 to 2 mm, more preferably 0.2 to 0.6 mm. For the purpose of enhancing the adhesive strength to the nonwoven fabrics 11 and 12, improving the spinnability into elastic filaments 13, and improving the stretch characteristics of the stretch sheet 10, the elastic filaments 13 may be conjugate filaments having, e.g., a side-by-side configuration, a sheath/core configuration, or a sea/island configuration. The conjugate filament is preferably composed of a PP-based elastomeric resin and a styrene elastomeric resin.

The spun, molten elastic filaments 13 are joined with a web of a first nonwoven fabric 11 and a web of a second nonwoven fabric 12 that are unrolled from the respective rolls and fed at equal speeds. The elastic filaments 13 as sandwiched are taken off at a predetermined speed, which is equal to the feed speed of the two webs. The take-off speed of the elastic filaments 13 influences the diameter and draw ratio of the elastic filaments 13. The tension of the elastic filaments 13 caused by the drawing prevents the elastic filaments 13 from being disturbed by airflow or static electricity generating in joining the elastic filaments 13 to the webs of the nonwoven fabrics 11 and 12, whereby the elastic filaments are successfully disposed to extend in one direction without intersecting. From this point of view, it is preferred that the take-off speed of the elastic filaments 13 be controlled with respect to the resin extrusion rate through the spinning nozzle orifices so as to result in a draw ratio of 1.1 to 400, more preferably 4 to 100, even more preferably 10 to 80.

The elastic filaments 13 are joined to the first and second nonwoven fabrics 11 and 12 before they solidify, that is, while they are in a fusion-bondable state. Therefore, the elastic filaments 13 are fusion bonded to the first and second nonwoven fabrics 11 and 12 as sandwiched therebetween. To put it another way, the elastic filaments 13 are drawn by take-off before they solidify while being fusion bonded to the moving nonwoven fabrics 11 and 12. Any external heat is applied to the first and second nonwoven fabrics 11 and 12 in achieving the fusion bonding of the elastic filaments 13. The fusion bonding of the elastic filaments 13 to the nonwoven fabrics 11 and 12 completes only by the heat of fusion of the fusion bondable elastic filaments 13. As a result, only the fibers present around each elastic filament 13 of the fibers constituting the nonwoven fabrics 11 and 12 are fusion bonded to the elastic filament 13, whereas more remote fibers are not fusion bonded. Thus, the heat applied to the nonwoven fabrics 11 and 12 is minimized, whereby the good feel essentially possessed by the nonwoven fabric itself is retained to provide the resulting stretch sheet 10 with a good feel.

The distance of travel (travelling distance) L of the spun molten elastic filament from the tip 16a of the spinning nozzle 16 until contact with the nonwoven fabrics 11 and 12, that is, the shortest distance between the spinning nozzle tip 16a and the contact points 11a and 12a at which the molten elastic filament 13 first comes into contact with the nonwoven fabrics 11 and 12 (see Fig. 6) is preferably 600 mm or shorter, more preferably 100 to 500 mm. The travelling distance L of the molten elastic filaments 13 being within 600 mm ensures the fusion bonding of the elastic filaments 13 before they solidify, which ensures producing a stretch sheet having interlaminar bonding strength and stretch characteristics in a good balance, namely, a stretch sheet having an elastic filament/nonwoven fabric bonding strength of 10 cN/25 mm or more and a 25% retraction strength per unit elastic resin content of 1.0 cN/ $\{50\ mm\cdot(g/m^2)\}$ or more. When the travelling distance L of the molten elastic filaments 13 is 100 mm or longer, the molten elastic filaments 13 are cooled to have an increased viscosity enough to be resistant to collapse between the nip rollers 18a and 18b, thereby achieving the recited preferred range of the average bonding ratio between the elastic filaments 13 and the fibers constituting the extensible nonwoven fabrics 11 and 12. When the travelling distance L of the molten elastic filaments 13 is 600 mm or shorter, the filaments are prevented from being disturbed by static electricity or airflow, resulting in reduced occurrences of adhesion or overlapping of the filaments to or on each other and filament breakage. Therefore, the upper limit of the travelling distance L is preferably 600 mm.

The travelling distance L is adjustable by the adjustment of the position of the spinning nozzles and/or the nip rollers. In the present embodiment, the fusion bonding of the elastic filaments 13 to the nonwoven fabrics 11 and 12 is accomplished by passing the molten elastic filaments 13 as spun from the spinning nozzles 16 and the moving webs of the nonwoven fabrics 11 and 12 between nip rollers 18a and 18b. The stretch sheet 10 of the present embodiment has the elastic filaments 13 bonded to the nonwoven fabrics 11 and 12 over the whole length thereof as previously described. To achieve this form of bonding, the present process of production employs cylindrical rollers with a smooth surface as the nip rollers 18a and 18b.

The spun, elastic filaments 13 are drawn to have the molecules oriented in the drawing direction and their diameter reduced until they are joined with the first and second nonwoven fabrics 11 and 12. As a result of the molecular orientation, there are obtained elastic filaments 13 having a small extension/retraction ratio of strength at 50% elongation. In order to sufficiently draw the elastic filaments 13 and to prevent breakage of the filaments 13 during the drawing operation, air at a prescribed temperature (hot air or cold air) may be blown to the spun elastic filaments 13 to control the temperature of the filaments 13.

The elastic filament 13 may be drawn either in its molten state (melt drawing) or in a softened state in the course of cooling. The term "molten state" means a state of a resin that flows when subjected to an outer force. The melting temperature of a resin is measured as a peak of tan$\delta$ obtained by viscoelasticity measurement (for example, an oscillatory strain is applied to a resin between parallel disks in the rotation direction). It is recommended to provide a sufficiently long drawing zone so as to avoid breakage of the filaments being drawn. From this viewpoint, the elastic resin preferably has a melting temperature of 130° to 300°C. In terms of heat resistance of the elastic resin, the melting temperature is preferably 220°C or lower. The molding temperature for forming the elastic filaments 13 (i.e., the die temperature) is preferably higher than the resin melting temperature by at least 50°C to increase the flowability of the resin thereby to improve molding properties. In terms of heat resistance, the molding temperature is preferably not higher than the resin melting temperature by more than 110°C The softening point is obtained as a Tg, one of the viscoelastic characteristics of the elastic resin as measured on a sheet-shaped specimen. By the term "softened state" is meant a state between the softening temperature and the melting temperature. A state at a temperature lower than the softening temperature is referred to as a solidified state. The elastic resin preferably has a softening temperature of 60°C or higher, more preferably 80° to 180°C, in view of elastic resin crystal growth during storage of the stretch sheet 10 and reduction in stretch characteristics of the stretch sheet 10 by a body temperature.

It is preferred that the elastic filaments 13 is joined to the webs of the nonwoven fabrics 11 and 12 at a temperature of 100°C or higher, more preferably 120°C or higher, even more preferably 140°C or higher, so as to ensure fusion bonding to the fibers. It is preferred for the elastic filaments 13 to have a temperature of 180°C or lower, more preferably 160°C or lower, thereby to retain their shape and to provide a stretch sheet 10 with good stretch characteristics. Accordingly, a preferred filament temperature ranges from 120° to 160°C, and a more preferred filament temperature ranges from 140° to 160°C. The temperature of the filament 13 being bonded (hereinafter referred to as a bonding temperature) is measurable by using, as a laminating material, film (e.g., of modified polyethylene or modified polypropylene)  having a melting point different from that of the elastic resin of the elastic filament and observing the bonding condition between the filament and the film. When the elastic filament and the laminating film are fusion bonded to each other, then the bonding temperature is at or above the melting point of the laminating film.

The elastic filament 13 is in a substantially nonstretched state (incapable of contraction when released from outer force) at the time of bonding to the nonwoven fabrics 11 and 12. In order to obtain sufficient bonding strength, it is preferred that, when the elastic filament 13 and the nonwoven fabrics 11 and 12 are joined to each other, at least part of the fibers constituting the nonwoven fabrics 11 and 12 is fused and bonded to the elastic filament. It is more preferred that both the elastic filament 13 and at least part of the fibers constituting the nonwoven fabrics 11 and 12 are fused and bonded to each other. The stretch characteristics of the resulting stretch sheet 10 are influenced by the density of the bonds between the elastic filaments 13 and the nonwoven fabrics 11 and 12. The stretch characteristics are controllable by the bonding temperature, bonding pressure, and debonding of the bonds by processing for elasticity development (described *infra*) of the nonwoven fabrics 11 and 12. Fusion bonding the constituent fibers of the nonwoven fabrics 11 and 12 to the elastic filaments 13 increases the strength of the individual bonds. It is advantageous to lower the bond density in that stretch hindrance by the nonwoven fabrics 11 and 12 is lessened and that the resulting stretch sheet 10 has sufficient bonding strength.

The elastic filaments 13 are joined to the first and second nonwoven fabrics 11 and 12 as arranged to extend in one direction in a non-intersection relation to each other. The nonwoven fabrics 11 and 12 thus having the elastic filaments 13 sandwiched therebetween are then passed between a pair of nip rollers 18. The pressing conditions affect the feel of the resulting stretch sheet 10. Too high a nip pressure causes the elastic filaments 13 to bite into the nonwoven fabrics 11 and 12, which can result in poor feel of the stretch sheet 10. In view of this, an excessively high nip pressure is not needed. Such a nip pressure that brings the elastic filaments 13 into contact with both the nonwoven fabrics 11 and 12 would be enough.

In another preferred mode of pressing the three members, i.e., the first and second nonwoven fabrics 11 and 12 having the elastic filaments 13 joined thereto and sandwiched therebetween, a clearance W (see Fig. 6) may be provided between the nip rollers 18a and 18b, through which the elastic filaments 13 and the nonwoven fabrics 11 and 12 are united. In order to obtain sufficient bonding strength without damaging the elastic filaments, the clearance W between the rollers 18a and 18b is preferably 0.05 to 1 mm, more preferably 0.05 to 0.5 mm.

Taking the sum of the thicknesses of the nonwoven fabrics 11 and 12 before being bonded and the thickness of the elastic filament immediately before being bonded (nipped) as measured in the nonwoven fabric thickness direction as T0, the ratio of the clearance W between the nip rollers 18a and 18b to T0 (W/T0) is preferably 0.05 to 0.8, more preferably 0.1 to 0.4. The thickness of the nonwoven fabric can be determined by the aforementioned method. The thickness (mm) of the elastic filament is obtained as $2\times\sqrt{(G/\rho vn\pi)}$, where G is an extrusion output per unit time (g/min) over the whole width of the machine, n is the number of filaments in the whole width of the machine, v is the velocity (m/min) of the nip rollers, and $\rho$ is the density (g/cm$^3$) of the filament resin. With W/T0 being in that range, the density of the bonds between the elastic filaments and the nonwoven fibers and the bite of the nonwoven fibers into the elastic filaments are well-balanced, whereby both the above described bonding strength between the elastic filaments and the nonwoven fabrics and the 25% retraction strength per unit resin content of the stretch sheet are obtained.

Also included in the pressing conditions of the nip rollers 18 is the temperature of the nip rollers 18. The inventors' study has revealed that a stretch sheet 10 with a better feel is obtained when the nip rollers 18 are not heated (or left to nature) or are positively cooled rather than when the nip rollers 18 are heated. Where the nip rollers 18 are cooled, the surface temperature of the nip rollers 18 is preferably set between 10°C and 50°C using a coolant, such as cooling water.

There is thus obtained a composite 19 having two nonwoven fabrics 11 and 12 and the elastic filaments 13 interposed therebetween. In the case when the nonwoven fabrics 11 and 12 are essentially extensible, the composite 19 is *per se* a stretch sheet 10. In the case when the nonwoven fabrics 11 and 12 are essentially inextensible, on the other hand, the composite 19 including the nonwoven fabrics 11 and 12 is subjected to processing for elasticity development in the extending direction of the elastic filaments 13 to impart stretchability to the composite 19. The processing is carried out in the present process by the use of an elasticity developing unit 22 having a pair of corrugating rollers 20 and 21 each having alternating ridges (teeth) and grooves (bottoms) along the circumferential direction thereof, thereby imparting stretchability to the composite 19 in the moving direction of the composite 19, i.e., the extending direction of the elastic filaments 13.

The elasticity developing unit 22 has a known vertical displacement mechanism (not shown) for vertically displacing the shaft of either one of or both of the corrugating rollers 20 and 21 to adjust the clearance between the rollers 20 and 21. In the present process, the corrugating rollers 20 and 21 are configured such that the ridges of the corrugating roller 20 fit with clearance into the spaces between every adjacent ridges of the other corrugating roller 21 and that the ridges of the other corrugating roller 21 fit with clearance into the spaces between every adjacent ridges of the corrugating roller 20. The composite 19 is introduced into the nip between the so configured corrugating rollers 20 and 21 to develop elasticity.

In the elasticity developing unit 22, each of the corrugating rollers 20 and 21 may be a drive roller attached to a motor, or either one of them may be a drive roller (and the other is a driven roller). In the present process, only the lower corrugating roller 21 is attached to a motor, while the upper corrugating roller 20 is not attached to a motor and is driven by the intermeshing drive roller 21. To use a driven roller is advantageous in that the high basis weight portions 14 and the low basis weight portions 15 easily form in clear stripes to provide an improved design and that the low basis weight portions 15 have a further decreased basis weight to provide an improved breathability. The corrugating rollers 20 and 21 may have a common involute or cycloid tooth profile. An involute or cycloid tooth profile with a reduced tooth thickness is particularly preferred.

Fig. 7 schematically illustrates the composite 19 being processed for elasticity development. While the composite 19 is passed between the corrugating rollers 20 and 21, the portions of the composite 19 that come into contact with the top surface of the ridges 23 and 24 of the corrugating rollers 20 and 21 (i.e., the portions between P3 and P2 and portions between P1 and P4, respectively), are little stretched. On the other hand, the portions of the composite 19 that are pressed by the ridgeline (tooth surface) of the ridges 24 of the drive corrugating roller 21 onto the ridgeline of the ridges 23 of the driven corrugating roller 20 (e.g., the portions between P2 and P1) are largely stretched by the corrugating rollers 20 and 21. The portions of the composite 19 that are separated away from the ridges 23 of the corrugating roller 20 by the tips of the ridges 24 of the corrugating roller 21 (i.e., the portions between P4 and P3) are also stretched largely while not so largely as the portions between P2 and P1.

Although the portions of the composite 19 in contact with the top surface of the ridges 23 and 24 of the corrugated rollers 20 and 21 (the portions between P3 and P2 and the portions between P1 and P4) are little stretched as stated, they become thinner because they are pressed radially, i.e., in the thickness direction of the composite 19 from one side. The portions between P3 and P2 and the portions between P1 and P4 are opposite in direction of thickness reduction because the directions of pressing are opposite to each other.

As a result of the above-described stretch processing, the nonwoven fabrics 11 and 12 of the composite 19 are stretched efficiently while preventing separation between the elastic filaments and the nonwoven fabrics 11 and 12 thereby to impart stretchability to the composite 19. The portions that are largely stretched (the portions between P2 and P1 and the portions between P4 and P3) become the low basis weight portions 15, while the portions that are stretched little (the portions between P3 and P2 and the portions between P1 and P4) become the high basis weight portions 14.

When, in particular, the nonwoven fabrics 11 and 12 contain low-drawn fibers, the low-drawn fibers will be drawn in the portions between P2 and P1 and the portions between P4 and P3 to form the varied thickness fibers with a periodically varied thickness.

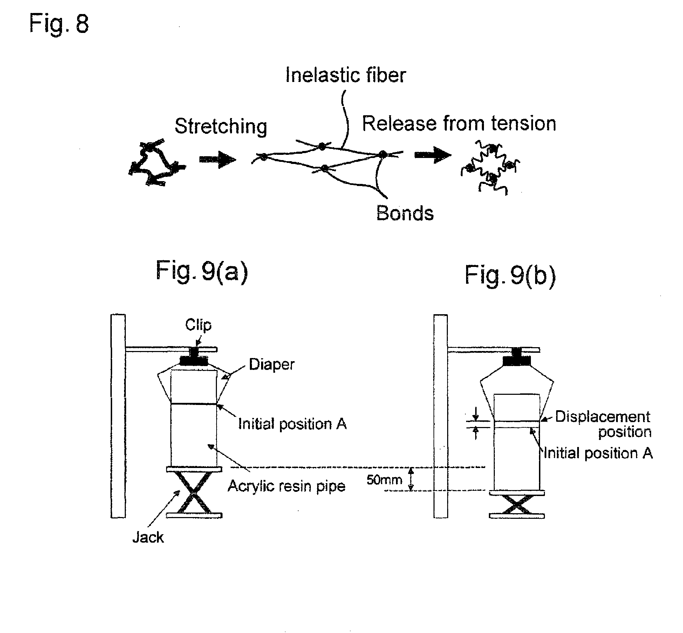

The stretching force by the corrugating rollers 20 and 21 is exerted primarily to draw the low-drawn fibers while preventing excessive force from being applied to the bonds between the nonwoven fabrics 11 and 12 and the elastic filaments 13. As a result, the composite 19 is efficiently stretched without causing destruction of the bonds or separation between the nonwoven fabrics 11 and 12 and the elastic filaments 13. As illustrated in Fig. 8, this stretch processing extends the nonwoven fabrics 11 and 12 sufficiently without destroying the interfiber bonds, whereby the interference by the nonwoven fabrics 11 and 12 with the free expansion and contraction of the elastic filaments 13 is greatly lessened. Thus, the process described accomplishes efficient production of the stretch sheet 10 exhibiting high strength and stretchability and a good appearance with little break or fuzzing. For the sake of convenience, the stretched inelastic fibers are depicted as having a uniform thickness in Fig. 8.

As described, in the case where the nonwoven fabrics 11 and 12 contain low-drawn fibers, the low-drawn fibers are successfully processed to develop elasticity without causing destruction of their interfiber bonds, so that reduction in strength of the nonwoven fabrics 11 and 12 due to the elasticity developing processing can be minimized.

Specifically, the ratio of the tensile strength of a stretch sheet 10 after the stretching operation to the tensile strength of a composite 19 before the stretching operation is preferably 0.3 to 0.99, more preferably 0.5 to 0.99, even more preferably 0.7 to 0.99, approaching to 1. The term "tensile strength" as used herein denotes a strength measured in accordance with the method of measuring maximum strength described below.

Method of measuring maximum strength:

A test specimen measuring 200 mm long along the stretch direction and 50 mm wide along the direction perpendicular to the stretch direction is cut out of a stretch sheet 10. The initial jaw separation is 150 mm. The specimen is elongated in the stretch direction at a rate of 300 mm/min while recording the load. The maximum load recorded was taken as a maximum strength. The maximum strength of the composite 19 before the elasticity developing processing is also determined in the same manner. The measurement is carried out in a measuring environment of 22°C and 65% RH preferably by using a tensile tester Autograph AG-1kNIS from Shimadz Corp.

The composite 19 is thus converted to a desired stretch sheet 10 by the elasticity developing processing between a pair of the corrugating rollers 20 and 21. After having passed through the corrugating rollers 20 and 21, the resulting stretch sheet 10 is rapidly released from the stretched state in the MD by virtue of its own retractability and returns almost to its original length in the MD. On being stretched again, the stretch sheet 10 has the high basis weight portions 14 and the low basis weight portions 15 alternating in the extending direction of the elastic filaments 13. When the resulting stretch sheet 10 is released from the stretched state, it may be released from the stretched state either completely or in a manner that the stretched state remains to some extent as long as extensibility and retractability develop.

By the above described processing for elasticity development, the thickness of the composite 19 is preferably increased to 1.1 to 4 times, more preferably 1.3 to 3 times, the thickness before the processing. By such processing, the fibers of the nonwoven fabrics 11 and 12 extend and become finer as a result of plastic deformation. At the same time, the nonwoven fabrics 11 and 12 become bulkier to provide a better feel to the touch and better cushioning.

The load measured when the stretch sheet 10 thus obtained is 100% elongated in the extending direction of the elastic filaments 13 and then 50% retracted (hereinafter referred to as a "50% retraction strength") is taken as load A, and the load measured when the stretch sheet 10 is 50% elongated in the extending direction of the elastic filaments 13 (hereinafter referred to as a "50% extension strength") is taken as load B. It is preferred for exhibiting sufficient stretch characteristics that the stretch sheet 10 to have a ratio of load A to load B (A/B) of at least 50%, more preferably 65% or higher.

The overall basis weight of the stretch sheet 10, while varying according to the intended use, is preferably 10 to 150 g/m$^2$, more preferably 25 to 60 g/m$^2$. The thickness of the stretch sheet 10 is preferably 0.05 to 5 mm, more preferably 0.5 to 2 mm. The thickness of the stretch sheet 10 is measured in the same manner as described with respect to the thickness of the nonwoven fabrics 11 and 12.

The stretch sheet 10 of the present embodiment is suited for use as an outer cover of a disposable pull-on diaper. For instance, the stretch sheet 10 of the present embodiment may be used as an outer sheet that is joined with an inextensible inner sheet to make the outer cover. It is also useful in other various applications, including disposable clothing for medical use, cleaning sheets, eye patches, masks, and bandages, taking advantage of its good feel, resistance to fuzzing, stretchability, breathability, and so on. It is especially suited for use as a component of absorbent articles, like sanitary napkins and disposable diapers. Examples of such a component include a fluid permeable sheet (e.g., topsheet and sublayer sheet) disposed on or above the skin facing side of an absorbent core, a sheet defining the exterior surface of a disposable diaper, a sheet used to elasticize a waist portion, a below-waist portion, a leg portion, and the like of a disposable diaper, a sheet used to form wings of a winged sanitary napkin, and a sheet used to elasticize any other portion of an absorbent article. The stretch sheet 10 may have a properly selected basis weight and thickness according to the intended use. For example, when used as a component of an absorbent article, a preferred basis weight is about 20 to 60 g/m$^2$, and a preferred thickness is about 0.5 to 1.5 mm.

While the present invention has been described based on its preferred embodiments, the invention is not limited to the embodiments. For example, while the stretch sheet 10 of the foregoing embodiments has a number of elastic filaments 13 held between two nonwoven fabrics 11 and 12, the stretch sheet may be composed of a single layer of nonwoven fabric and a number of elastic filaments bonded to the nonwoven fabric. In this modification, it is preferred to use a resin having low surface tack in a solidified state as a raw material of the elastic filaments so as to prevent blocking while the stretch sheet 10 is stored in roll form.

In the foregoing embodiments since all the elastic filaments 13 are equal in diameter and are arranged at a constant pitch, every part of the stretch sheet 10 exhibits equal extension stress. Alternatively, the stretch sheet may be configured to have two or more zones different in extension stress in the extending direction of the elastic filaments. Such two or more zones are in substantially parallel relationship with respect to the extending direction of the elastic filaments. The extension stress may be varied from zone to zone by varying the distance between the elastic filaments and/or the diameter of the elastic filaments. The extension stress difference between different zones may also be created by introducing two or more different resins to predetermined respective spinning nozzles.

The stretch sheet 10 may be embossed in part. The elastic filaments 13 may be cut or heat-sealed in part. These operations are selected for the purpose of making a desired part of the stretch sheet 10 non-stretchable, increasing the strength of a desired part of the stretch sheet 10, laminating the stretch sheet 10 with other members, or as a matter of design choice.

The direction of stretch in the elasticity developing processing following bonding the elastic filaments to the nonwoven fabrics 11 and 12 is not limited to the machine direction and may be, for example, oblique to the machine direction. Two or more different manners of elasticity developing processing may be combined, or the stretch ratio may be increased stepwise during the elasticity developing processing, or the elasticity developing processing may be done in part of the composite 19. The direction of the elasticity developing processing is not limited to one direction and may be mutually perpendicular directions. The first and second nonwoven fabrics may be combined such that the stretch direction of one of them is perpendicular to that of the other to produce a stretch sheet having stretchability in every direction.

The elasticity developing unit equipped with a pair of corrugating rollers 20 and 21 as used in the process of the foregoing embodiment may be replaced with an elasticity developing unit equipped with a tenter.

The step of bonding the elastic filaments 13 to the nonwoven fabrics 11 and 12 in the above described process of production may be carried out by directly extruding the elastic filaments 13 on one of the nonwoven fabrics without involving melt drawing. In this case, the draw ratio is 1. An adhesive may be applied to the nonwoven fabrics 11 and 12 or the elastic filaments 13 before bonding them as an auxiliary bonding means, followed by bonding the elastic filaments in a substantially undrawn state. After the nonwoven fabrics 11 and 12 and the elastic filaments 13 are joined together without the aid of an adhesive, the resulting laminate may be subjected to heat treatment (e.g., hot air blowing in a through-air system, steam jet treatment or heat embossing) or mechanical entanglement (e.g., needle punching or hydroentanglement) as an auxiliary bonding means. In this case, a fiber web may take the place of the nonwoven fabric on one or both sides.

Examples

The present invention will now be illustrated in greater detail by way of Examples, but it should be understood that the invention is not limited thereto.

Example 1-1

A stretch sheet shown in Figs. 1 and 2 was produced by the use of the apparatus illustrated in Fig. 5. Air-through nonwoven fabric having a basis weight of 15 g/m$^2$ was used as a first and a second nonwoven fabric 11 and 12. The fibers fabricating the nonwoven fabric were sheath/core conjugate fibers (PE sheath, PET core) having a diameter of 19 $\mu$m, a maximum elongation of 180%, and a fiber length of 44 mm. An SEPS resin elastomer (weight average molecular weight: 50, 000; MFR: 60 g/10 min (230°C, 2.16 kg, JIS K-7210:1999) was used as a material of elastic filaments 13. The spinning conditions were: 310°C in temperature of the spinning head 17, 400 $\mu$m in diameter of spinning nozzles 16, and 1 mm in pitch of the spinning nozzles 16. The elastic filaments 13 had a diameter of 120 $\mu$m, an apparent weight per unit area (the weight of the filaments in a sample/the area of the sample sheet) of 10 g/m$^2$, and a draw ratio of 11. The draw ratio is defined as (nozzle orifice diameter/filament diameter before elasticity developing processing) $^2$. Elasticity developing processing of a composite 19 was performed using an elasticity developing unit 22 equipped with a pair of corrugating rollers 20 and 21 each having alternating ridges and grooves running in the axial direction. The pitches of the ridges and the grooves of each corrugating roller were both 2.0 mm. The pitch P of adjacent ridges of the intermeshing corrugating rollers was 1.0 mm. The depth of engagement of the corrugating rollers was adjusted so that the composite 19 was stretched in the extending direction of the elastic filaments to a stretch ratio of 3.0 to develop elasticity. There was thus obtained a stretch sheet 10 stretchable in the extending direction of the elastic filaments 13 and having a basis weight of 40 g/m$^2$. The resulting stretch sheet 10 assumed a stripe pattern of the elastic filaments 13 through the nonwoven fabric layers. The stretch sheet 10 also showed a stripe pattern formed of alternating high basis weight portions and low basis weight portions. These two stripe patterns, being superimposed on each other, provided the stretch sheet 10 with a grid pattern. The cross-section of the elastic filaments 13 in the stretch sheet 10 was oval, having a major axis in a planar direction of the sheet 10 and a major to minor axis ratio of 1.6. When stretched 1.5 times the original length, the sheet 10 reduced in width to 96% of the original width, i.e., the width contraction was 4%. Microscopic observation of the fibers constituting the nonwoven fabrics 11 and 12 in the stretch sheet 10 revealed that their thickness was not uniform along their length. The fibers were found to have a maximum diameter of 26.1 $\mu$m and a minimum diameter of 6.8 $\mu$m giving a diameter ratio of 3.8 and had their thickness varied periodically within that range.

Example 1-2

A stretch sheet 10 was obtained in the same manner as in Example 1-1 with the following exceptions. The spinning nozzles 16 were arranged at a pitch of 1 mm in the cross-machine direction of the spinning head 17 to form a first row of nozzles. A second row of nozzles having the same pitch as the first row was formed 1 mm apart from the first row in the machine direction of the spinning head 17. The first and the second rows of nozzles were offset by half the pitch of the nozzles. The draw ratio of the elastic filaments 13 was changed to 25 to reduce the diameter to 80 $\mu$m. The pitch of the elastic filaments 13 in the stretch sheet 10 was 0.5 mm.

Example 1-3

A stretch sheet 10 was obtained in the same manner as in Example 1-1, except that the drawing of the elastic filaments 13 was performed under different conditions to form a first zone and a second zone in a side by side relationship in the width direction of the stretch sheet 10. The first zone was made by using spinning nozzles 16 having an orifice diameter of 400 $\mu$m and drawing the elastic filaments 13 at a draw ratio of 11 to reduce the diameter to 120 $\mu$m. The second zone was made by using spinning nozzles 16 having an orifice diameter of 300 $\mu$m and drawing the elastic filaments 13 at a draw ratio of 18 to reduce the diameter to 70 $\mu$m. The diameter of the elastic filaments was measured in each of the two zones.

Example 1-4

A stretch sheet 10 was obtained in the same manner as in Example 1-1, except that the styrene elastomer used in Example 1-1 was dry-blended with 1.5 wt% purple-colored master batch compound (PE resin base, pigment concentration: 60 wt%) to form purple elastic filaments 13. The resulting stretch sheet 10 displayed a clearer stripe pattern of the elastic filaments 13 through the nonwoven fabrics.

Example 1-5

The composite 19 used in Example 1-2 was heat treated by blowing hot air in a through-air system and then subjected to stretch processing under the same conditions as in Example 2 to obtain a stretch sheet 10. The stretch sheet exhibited an increased bonding strength owing to interfiber fusion bonding in the nonwoven fabrics 11 and 12.

Comparative Example 1-1

A stretch sheet was prepared in accordance with the process of patent document 1.
A stretch net available from Conwed Plastic Company (basis weight: 73 g/m$^2$) was used. Through-air nonwoven fabrics having a basis weight of 20 g/m$^2$ was used as a first and a second nonwoven fabric 11 and 12. The fibers of the nonwoven fabric were sheath/core conjugate fibers (PE sheath/PET core) having a diameter of 17 $\mu$m, a maximum elongation of 30%, and a length of 44 mm. The grid pattern of the stretch net was generally composed of 3.2 mm squares and was disposed with the constituting strands parallel in the MD and CD of the nonwoven fabrics 11 and 12. The three layers were heat/pressure-bonded on a metal press of which the pressure plates were heated to about 120°C and about 60°C under a pressure of about 10 kg/cm$^2$ for 10 seconds. The resulting composite sheet was subjected to processing for elasticity development in a stretching apparatus (elasticity developing unit) equipped with a pair of intermeshing corrugating rollers having axially alternating large-diameter segments and small-diameter segments. The pitch P of adjacent large-diameter segments of the intermeshing corrugating rollers was 1.0 mm. When the processing was effected to a high stretch ratio, the nonwoven fabrics tended to make a hole on account of lack of extensibility of the nonwoven fibers. Then, the processing was carried out at a stretch ratio of 2.7 in the MD. The degree of stretching was controlled by the adjustment of the depth of engagement between the upper and the lower corrugating rollers. When stretched, the resulting stretch sheet underwent considerable contraction in width because of its low stretchability. It was inferior in feel due to the stiffness of the elastic net.

Comparative Example 1-2

A stretch sheet was obtained using a stretch net from Conwed Plastic Company (basis weight: 55 g/m$^2$) in the same manner as in Comparative Example 1-1. The grid pattern of the net was generally composed of rhombi with side lengths of 9.5 mm and was disposed with the diagonals of the rhombi in parallel with the MD and CD of the nonwoven fabrics 11 and 12. The three layers were heat/pressure-bonded on a metal press of which the pressure plates were heated to about 120°C and about 60°C under a pressure of about 10 kg/cm$^2$ for 10 seconds. The resulting composite sheet was stretched 3.0 times in the MD by means of a stretching apparatus (elasticity developing unit) equipped with a pair of intermeshing corrugating rollers having axially alternating large-diameter segments and small-diameter segments. The pitch P of adjacent large-diameter segments of the intermeshing corrugating rollers was 1.0 mm. As a result of this elasticity developing processing, a number of holes were made in the nonwoven fabrics due to the inextensibility of the nonwoven fibers. The resulting stretch sheet, while bulky as a whole, was inferior in feel due to the hard bonds between the elastic net and the nonwoven fabrics.

Evaluation

The stretch sheets obtained in Examples and Comparative Examples were evaluated for 50% retraction strength/50% extension strength and feel in accordance with the following test methods. The thicknesses of the stretch sheet and the nonwoven fabrics were measured by the above described method. The results obtained are shown in Table 1 below.

(1) 50% Retraction strength/50% extension strength

A test specimen measuring 200 mm long along the stretch direction and 50 mm wide along the direction perpendicular to the stretch direction was cut out of the stretch sheet. The specimen was set on a tensile tester Autograph AG-1kNIS from Shimadz Corp. at an initial jaw separation of 150 mm. The specimen was elongated in the stretch direction at a rate of 300 mm/min. The load recorded at 50% extension was read as a 50% extension strength. The specimen was further elongated up to 100% elongation and then retracted to 50% elongation in the opposite direction at the same  rate. The load at this point was read as a 50% retraction strength. The measurement was made in a measuring environment of $20\pm2$°C and $65\pm5$% RH.

(2) Feel

A panel consisting of 10 female panelists touched the stretch sheet placed in a dark box so that they were unable to see the stretch sheet in an environment of 25°C and 40% RH and scored the feel in accordance with the following scale. The scores assigned by the 10 panelists were averaged to give a feel value. The average was rounded to the nearest whole number.
5=Good; 4=fairly good; 3=medium; 2=slightly poor; 1=poor
Table 1

| | | Example | | | | | Comp. Example | |
|---|---|---|---|---|---|---|---|---|
| | | 1-1 | 1-2 | 1-3 | 1-4 | 1-5 | 1-1 | 1-2 |
| Spinning Nozzle: | Diameter ($\mu$m) | 400 | 400 | 400/300 | 400 | 400 | - | - |
| | Pitch (mm) | 1 | 0.5 | 1 | 1 | 1 | - | - |
| Elastic Filament | Draw Ratio | 11 | 25 | 11/18 | 11 | 11 | - | - |
| | Average Diameter ($\mu$m) | 120 | 80 | 120/70 | 120 | 120 | 400/320 | 400/300 |
| | Diameter in Thickness Direction ($\mu$m) | 92 | 70 | 92/61 | 92 | 92 | 110/110 | 95/80 |
| | Average Flatness | 1.6 | 1.3 | 1.6/1.3 | 1.6 | 1.6 | 8.3/5.4 | 9.5/10 |
| Basis Weight (g/m$^2$) | 1st Nonwoven Fabric | 15 | 15 | 15 | 15 | 15 | 20 | 20 |
| | 2nd Nonwoven Fabric | 15 | 15 | 15 | 15 | 15 | 20 | 20 |
| | Stretch Sheet | 40 | 40 | 40/33 | 40 | 40 | 98 | 93 |
| 50% Retraction Strength/50% Extension Strength (%) | | 75 | 78 | 65/69 | 65 | 68 | 16 | 32 |

(continued)

|  |  | Example |  |  |  |  | Comp. Example |  |
|---|---|---|---|---|---|---|---|---|
|  |  | 1-1 | 1-2 | 1-3 | 1-4 | 1-5 | 1-1 | 1-2 |
| Thickness (mm) | Stretch Sheet | 1.3 | 1.3 | 1.3 | 1.3 | 1.1 | 1.6 | 1.8 |
|  | 1st Nonwoven Fabric | 0.6 | 0.6 | 0.6 | 0.6 | 0.5 | 0.7 | 0.8 |
|  | 2nd Nonwoven Fabric | 0.6 | 0.6 | 0.6 | 0.6 | 0.5 | 0.7 | 0.8 |
| Feel of Stretch Sheet |  | 5 | 5 | 5 | 5 | 5 | 2 | 2 |
| Width Contraction (%) |  | 4 | 4 | 3 | 4 | 4 | 20 | 36 |

As is apparent from the results in Table 1, the stretch sheets of Examples proved to have good stretch characteristics, to be bulky, and to have a good feel. The elastic filaments in each of them were observed to be in a spaced non-intersection relationship to each other. The stretch sheet of Comparative Example 1-1 was not good in appearance because the elastic strands of the net were collapsed by the pressing and therefore stood out and also because the net was glossy.

Examples 2-1 to 2-5 and Comparative Examples 2-1 to 2-3

A composite 19 (a precursor of a stretch sheet 10 shown in Figs. 1 and 2) was prepared by use of the apparatus illustrated in Fig. 5.
The apparatus included a spinning head 17 connected to a single-screw extruder. The spinning head 17 had 250 cylindrical spinning nozzles 16 each having an inner diameter of 0.45 mm and a length of 4.5 mm linearly arranged at an interval of 1 mm. Each of triblock copolymers (1) to (8) below was melt-spun from the nozzles 16 at a resin temperature of 290°C and an output of 0.3 kg/min into filaments (precursors of elastic filaments 13). The spun filaments were sandwiched in between facing webs of air-through nonwoven fabrics 11 and 12 at a position 100 mm below the tip of the spinning nozzle 16 to obtain a composite 19 having the elastic filaments 13 held between the nonwoven fabrics 11 and 12. The nonwoven fabrics 11 and 12 each had a basis weight of 20 g/m$^2$, a thickness of 0.4 mm, and a fiber fineness of 3 dtex and were fed at a rate of 150 m/min. Both the nonwoven fabrics 11 and 12 were essentially inextensible.
Of the eight triblock copolymers (1) through (8) having an A1-B-A2 configuration, (1) to (5) are in the scope of the present invention, while (6) to (8) are out of the scope of the invention.

A1-B-A2 Triblock copolymer (1)

Eighty percent by weight of an A1'-B'-A2' triblock copolymer (SEPS triblock copolymer; styrene content: 30 wt%; ethylene-propylene content: 70 wt%; weight average molecular weight (Mw) of A1' and A2': 7500; Mw of B: 35000) and 20 percent by weight of an A1"-B"-A2" triblock copolymer (SEPS triblock copolymer; styrene content: 18 wt%; ethylene-propylene content: 82 wt%; Mw of A1" and A2": 8100; Mw of B": 73800) were blended in a Henschel mixer to provide a triblock copolymer (1). The triblock copolymer (1) had a maximum melt tension of 0.07 cN. As above explained, a maximum melt tension is defined to be the maximum value read when a resin composition in a molten state at 245°C is extruded from an orifice of a cylindrical nozzle of 0.45 mm in inner diameter and 4.5 mm in length at an extrusion rate of 8.4 m/min.
The Mw of the polymer block A1 in the triblock copolymer (1) was 7620. The Mw of the polymer block A1 is defined to be [(Mw of polymer block A1') x(rate of content of A1'-B'-A2' triblock copolymer in the total of two or more kinds of A1-B-A2 triblock copolymers in the resin composition) +(Mw of polymer block A1") x(rate of content of A1"-B"-A2" triblock copolymer in the total of two or more kinds of A1-B-A2 triblock copolymers in the resin composition)].
The Mw of the polymer block B in the triblock copolymer (1) was 42760. The Mw of the polymer block B is defined to be [(Mw of polymer block B') x(rate of content of A1'-B'-A2' triblock copolymer in the total of two or more kinds of A1-B-A2 triblock copolymers in the resin composition) +(Mw of polymer block B") x(rate of content of A1"-B"-A2" triblock copolymer in the total of two or more kinds of A1-B-A2 triblock copolymers in the resin composition)].
The polymer block A2 in the triblock copolymer (1) had an Mw of 7620. The Mw of the polymer block A2 is defined to be [(Mw of polymer block A2') x(rate of content of A1'-B'-A2' triblock copolymer in the total of two or more kinds of A1-B-A2 triblock copolymers in the resin composition) +(Mw of polymer block A2") x(rate of content of A1"-B"-A2" triblock copolymer in the total of two or more kinds of A1-B-A2 triblock copolymers in the resin composition)].

A1-B-A2 Triblock copolymer (2)

An SEPS triblock copolymer (styrene content: 18 wt%; ethylene-propylene content: 82 wt%; Mw of A1 and A2: 8100; Mw of B: 73800) was used as a triblock copolymer (2). The triblock copolymer had a maximum melt tension of 0.13 cN.

A1-B-A2 Triblock copolymer (3)

An SEBS triblock copolymer (styrene content: 20 wt%; ethylene-butylene content: 80 wt%; Mw of A1 and A2: 9400; Mw of B: 75200) was used as a triblock copolymer (3). The triblock copolymer (3) had a maximum melt tension of 0.15 cN.

A1-B-A2 Triblock copolymer (4)

Sixty percent by weight of an SEPS triblock copolymer (styrene content: 30 wt%; ethylene-propylene content: 70 wt%; Mw of A1' and A2': 7500; Mw of B': 35000) as an A1'-B'-A2' triblock copolymer and 40% by weight of an SEPS triblock copolymer (styrene content: 18 wt%; ethylene-propylene content: 82 wt%; Mw of A1" and A2": 8100; Mw of B": 73800) as an A1"-B"-A2" triblock copolymer were blended in a Henschel mixture to provide a triblock copolymer (4). The triblock copolymer (4) had a maximum melt tension of 0.20 cN.

The Mw of the polymer block A1 in the triblock copolymer (4) was 7740. The Mw of the polymer block A1 is defined to be [(Mw of polymer block A1') x(rate of content of A1'-B'-A2' in the total of two or more kinds of A1-B-A2 triblock copolymers in the resin composition) +(Mw of polymer block A1") x(rate of content of A1"-B"-A2" in the total of two or more kinds of A1-B-A2 triblock copolymers in the resin composition)].

The Mw of the polymer block B in the triblock copolymer (4) was 50520. The Mw of the polymer block B is defined to be [(Mw of polymer block B') x(rate of content of A1'-B'-A2' in the total of two or more kinds of A1-B-A2 triblock copolymers in the resin composition) +(Mw of polymer block B") x(rate of content of A1"-B"-A2" in the total of two or more kinds of A1-B-A2 triblock copolymers in the resin composition)].

The Mw of the polymer block A2 in the triblock copolymer (4) was 7740.

The Mw of the polymer block A2 is defined to be [(Mw of polymer block A2') x(rate of content of A1'-B'-A2' in the total of two or more kinds of A1-B-A2 triblock copolymers in the resin composition) +(Mw of polymer block A2") x(rate of content of A1"-B"-A2" in the total of two or more kinds of A1-B-A2 triblock copolymers in the resin composition)].

A1-B-A2 Triblock copolymer (5)

Fifty percent by weight of an SEPS triblock copolymer (styrene content: 30 wt%; ethylene-propylene content: 70 wt%; Mw of A1' and A2': 7500; Mw of B': 35000) as an A1'-B'-A2' triblock copolymer and 50% by weight of an SEPS triblock copolymer (styrene content: 30 wt%; ethylene-propylene content: 70 wt%; Mw of A1" and A2": 10500; Mw of B": 49000) as an A1"-B"-A2" triblock copolymer were blended in a Henschel mixture to provide a triblock copolymer (5). The triblock copolymer (5) had a maximum melt tension of 0.07 cN.

The Mw of the polymer block A1 in the triblock copolymer (5) was 9000. The Mw of the polymer block A1 is defined to be [(Mw of polymer block A1') x(rate of content of A1'-B'-A2' in the total of two or more kinds of A1-B-A2 triblock copolymers in the resin composition) +(Mw of polymer block A1") x(rate of content of A1"-B"-A2" in the total of two or more kinds of A1-B-A2 triblock copolymers in the resin composition)].

The Mw of the polymer block B in the triblock copolymer (5) was 42000. The Mw of the polymer block B is defined to be [(Mw of polymer block B') x(rate of content of A1'-B'-A2' in the total of two or more kinds of A1-B-A2 triblock copolymers in the resin composition) +(Mw of polymer block B") x(rate of content of A1"-B"-A2" in the total of two or more kinds of A1-B-A2 triblock copolymers in the resin composition)].

The Mw of the polymer block A2 in the triblock copolymer (5) was 9000. The Mw of the polymer block A2 is defined to be [(Mw of polymer block A2') x(rate of content of A1'-B'-A2' in the total of two or more kinds of A1-B-A2 triblock copolymers in the resin composition) +(Mw of polymer block A2") x(rate of content of A1"-B"-A2" in the total of two or more kinds of A1-B-A2 triblock copolymers in the resin composition)].

A1-B-A2 Triblock copolymer (6)

An SEPS triblock copolymer (styrene content: 30 wt%; ethylene-propylene content: 70 wt%; Mw of A1 and A2: 10500; average molecular weight of B: 49000) was used as a triblock copolymer (6). The triblock copolymer (6) had a maximum melt tension of 0.08 cN.

A1-B-A2 Triblock copolymer (7)

An SEBS triblock copolymer (styrene content: 20 wt%; ethylene-butylene content: 80 wt%; Mw of A1 and A2: 4750; average molecular weight of B: 38000) was X used as a triblock copolymer (7). The triblock copolymer (7) had a maximum melt tension of 0.05 cN.

A1-B-A2 Triblock copolymer (8)

An SEPS triblock copolymer (styrene content: 30 wt%; ethylene content: 70 wt%; Mw of A1 and A2: 22750; average molecular weight of B: 84500) was used as a triblock copolymer (8). The melt tension of the copolymer (8) was unmeasurable by the method illustrated in Fig. 4 because of a failure to extrude due to its high viscosity.

Evaluation

The characteristics of the elastic filaments 13 in the composites 19 prepared in Examples and Comparative Examples are shown in Tables 2 and 3 below together with the composition of the resin compositions used to make the elastic filaments 13. The characteristic qualities in Tables 2 and 3 were evaluated as follows.

(1) Spinnability into elastic filaments

Each of the resin compositions (triblock copolymers (1) to (8)) was melted at 245°C and extruded through a cylindrical nozzle having an orifice inner diameter of 0.45 mm and a length of 4.5 mm at an extrusion rate of 8.4 m/min into a filament, which was deposited on a stainless plate 100 mm below the nozzle orifice. A filament which was continuous without disruption and was observed to have a smooth side surface under a microscope at 200X was rated good in spinnability. A filament which was discontinuous due to breakage or non-uniform in thickness due to die swell was rated poor.

(2) Freedom from filament vibration

The elastic members in each of the composites obtained in Examples and Comparative Examples were observed through the nonwoven fabric with the naked eye to see if they vibrated over a sampling length of 30 cm in the MD (i.e., the moving direction of the composite in the production thereof). A composite in which less than eleven out of a hundred adjacent filaments intersected or contacted with each other was rated good, and a composite in which more than ten out of a hundred adjacent filaments intersected or contacted with each other was rated poor.

(3) Resistance of diaper against sliding down

Each of the composites obtained in Examples and Comparative Examples was stretched by using a stretching unit equipped with a pair of corrugating rollers illustrated in Fig. 7 to prepare a stretch sheet illustrated in Figs. 1 and 2. A disposable pull-on diaper having a circumference of 35 cm at the waist was made using the stretch sheet as a material of an outer cover. As illustrated in Fig. 9, the diaper was put upside down over an acrylic resin pipe having a circumference of 44 cm, and the lower part (crotch portion) of the diaper was secured with a clip. The acrylic resin pipe was placed on the platform of a jack. The pipe was marked at the position of the waist of the diaper (initial position A). With the diaper secured with the clip, the platform of the jack was lowered by 50 mm. The distance between the position of the waist after lowering the jack platform (displacement position) and the initial position A was measured. This distance was taken as an amount of displacement due to sliding of the diaper. The resistance of the diaper against sliding down was rated based on the amount of displacement according to the following standards.

Very good: The amount of displacement is less than 10 mm.
Good: The amount of displacement is 10 mm or more and less than 20 mm.
Fair (non-problematic for practical use): The amount of displacement is 20 mm or more and less than 30 mm.
Poor: The amount of displacement is 30 mm or more.

Abbreviations in Tables 2 and 3 have the following meanings: PS=polystyrene; P(E-P) =poly(ethylene-propylene); P(E-B) =poly(ethylene-butylene); Mw=weight average molecular weight; ND=not determined.

Table 2

| | Example 2-1 | Example 2-2 | Example 2-3 | Example 2-4 | Example 2-5 |
|---|---|---|---|---|---|
| A1-B-A2 Triblock Copolymer | (1)*1 | (2) | (3) | (4)*2 | (5)*3 |
| Kind of A1 | PS | PS | PS | PS | PS |
| Mw of A1 | 7620 | 8100 | 9400 | 7740 | 9000 |
| Kind of B | P(E-P) | P(E-P) | P(E-B) | P(E-P) | P(E-P) |
| Mw of B | 42760 | 73800 | 75200 | 50520 | 42000 |
| Kind of A2 | PS | PS | PS | PS | PS |
| Mw of A2 | 7620 | 8100 | 9400 | 7740 | 9000 |
| Sum of Mw of A 1 and Mw of A2 | 15240 | 16200 | 18800 | 15480 | 18000 |
| Max. Melt tension (cN) | 0.07 | 0.13 | 0.15 | 0.20 | 0.07 |
| Spinnability | good | good | good | good | good |
| Freedom of Filament Vibration | good | good | good | good | good |
| Resistance to Sliding down | very good | fair | fair | good | very good |
| *1 to *3: Mixture of two kinds of A1-B-A2 triblock copolymers | | | | | |

Table 3

| | Comparative Example 2-1 | Comparative Example 2-2 | Comparative Example 2-3 |
|---|---|---|---|
| A1-B-A2 Triblock Copolymer | (6) | (7) | (8) |
| Kind of A1 | PS | PS | PS |
| Mw of A1 | 10500 | 4750 | 22750 |
| Kind of B | P(E-P) | P(E-B) | P(E-P) |
| Mw of B | 49000 | 38000 | 84500 |
| Kind of A2 | PS | PS | PS |
| Mw of A2 | 10500 | 4750 | 22750 |
| Sum of Mw of A1 and Mn of A2 | 21000 | 9500 | 45500 |
| Max. Melt tension (cN) | 0.08 | 0.05 | ND*4 |
| Spinnability | poor (non-uniform, filament breakage) | poor (filament breakage) | ND*4 |
| Freedom of Filament Vibration | poor | poor | ND*4 |
| Resistance to Sliding down | fair | poor | ND*4 |
| *4: Failure to spin into elastic filaments due to high viscosity of triblock copolymer (8) | | | |

As is apparent from the results in Tables 2 and 3, Examples 2-1 through 2-5 using resin compositions within the scope of the invention exhibit excellent spinnability into filaments and no vibration of filaments in the stretch sheet, and provide diapers resistant to sliding down. In contrast, Comparative Example 2-1 fails to obtain continuous filaments because the spun filaments have non-uniform thickness due to die swell and break at extremely thin parts thereof. Comparative Example 2-2 also fails to obtain continuous filaments because the spun filaments, though having a uniform thickness, have a low melt tension. As a result, the diapers using the stretch sheets of Comparative Examples 2-1 and 2-2 have poor resistance against sliding down because of reduction in stretch characteristics due to the breakage of the filaments. Comparative Example 2-3 fails to spin the resin material into filaments due to too high of a viscosity of the triblock copolymer (8).

Example 3-1

A stretch sheet shown in Figs. 1 and 2 was prepared by use of the apparatus illustrated in Fig. 5. Air-through nonwoven fabric having a basis weight of 20 g/m$^2$ and a fiber density of 0.074 g/cm$^3$ was used as a first and a second nonwoven fabric 11 and 12. The fibers fabricating the nonwoven fabric were sheath/core conjugate fibers (PE sheath, PET core) having a diameter of 19 $\mu$m, a maximum elongation of 180%, and a fiber length of 44 mm. An SEPS resin elastomer (Mw: 50, 000; MFR: 60 g/10 min (230°C, 2.16 kg) was used as a material of elastic filaments 13. The spinning conditions were: 310°C in temperature of the spinning head 17, 400 $\mu$m in diameter of spinning nozzles 16, 1 mm in pitch of the spinning nozzles 16, and 11 in draw ratio. The elastic filaments 13 had a diameter of 107 $\mu$m. The weight of the filaments per unit area of the stretch sheet was 8 g/m$^2$. The nip rollers for bonding the elastic filaments to the nonwoven fabrics 11 and 12 were configured to have a clearance W of 0.2 mm. One of the nip rollers was a smooth metal roller, and the other was a rubber roller. The stretch processing of the composite 19 was performed using a stretching unit 22 equipped with a pair of corrugating rollers 20 and 21 each having alternating ridges and grooves running in the axial direction. The pitches of the ridges and the grooves of each corrugating roller were both 2.0 mm. The pitch P of adjacent ridges of the intermeshing corrugating rollers was 1.0 mm. The depth of engagement of the corrugating rollers was adjusted so that the composite 19 was stretched in the extending direction of the elastic filaments to a stretch ratio of 3.0. There was thus obtained a stretch sheet 10 stretchable in the extending direction of the elastic filaments 13 and having a basis weight of 48 g/m$^2$. The resulting stretch sheet 10 assumed a stripe pattern of the elastic filaments 13 through the nonwoven fabric layers. The stretch sheet 10 also showed a stripe pattern formed of alternating high basis weight portions and low basis weight portions. These two stripe patterns, being superimposed on each other, provided the stretch sheet 10 with a grid pattern. The cross-section of the elastic filaments 13 in the stretch sheet 10 was oval, having a major axis in a planar direction of the sheet 10 and a major to minor axis ratio of 1.5. When the stretch sheet 10 was stretched 1.5 times the original length, the width contraction was 4%.

Examples 3-2 to 3-4

A stretch sheet 10 was obtained in the same manner as in Example 3-1, except that the clearance W between the nip rollers was changed as indicated in Tables 4 and 5.

Examples 3-5 to 3-7

A stretch sheet 10 was obtained in the same manner as in Example 3-1, except that the distance from the tip of the spinning nozzles to the point of joining as shown in Tables 4 and 5 and that the nip rollers were set with no clearance therebetween.

Comparative Example 3-1

A stretch sheet was obtained in the same manner as in Example 3-1, except that an elastic resin film extruded from a T-slot die was used in place of the elastic filaments. Kraton G1657 (trade name, from Kraton Polymer) was used as an elastic resin.

Evaluation

The stretch sheets obtained in Examples and Comparative Example were measured or evaluated for the properties listed in Tables 4 and 5, of which "interlaminar peel strength" (the bonding strength between elastic filaments and nonwoven fabrics), "25% retraction strength (cN/ {50 mm·(g/m$^2$)} " (25% retraction strength per unit content of elastic resin in stretch sheet), "average bonding ratio between elastic filaments and nonwoven fibers", "width contraction (%) " (width contraction when stretch sheet is stretched 1.5 times), and thicknesses of stretch sheet and nonwoven fabrics were measured in accordance with the method previously described; "25% retraction strength (cN/50 mm) ", "25% retraction strength/25% extension strength (%) ", and "feel" were measured or evaluated as described below; and "intersection of elastic filaments" was observed with the naked eye. A stretch sheet having few intersections of the elastic filaments is given the highest grade. The more the intersections, the lower the grade.

(1) 25% Retraction strength/25% extraction strength and 25% retraction strength:

A test specimen measuring 200 mm long along the stretch direction and 50 mm wide along the direction perpendicular to the stretch direction was cut out of the stretch sheet. The specimen was set on a tensile tester Autograph AG-IkNIS from Shimadz Corp. at an initial jaw separation of 150 mm. The specimen was elongated in the stretch direction at a rate of 300 mm/min. The load recorded at 25% extension was read as a 25% extension strength. The specimen was further elongated up to 50% elongation and then retracted to 25% elongation in the opposite direction at the same rate. The load at this point was read as a 25% retraction strength. A 25% retraction strength/25% extension strength (%) was calculated from the readings. The measurement was made in a measuring environment of $20\pm2°C$ and $65\pm5\%$ RH.

(2) Feel

A panel consisting of 10 female panelists touched the stretch sheet placed in a dark box so that they were unable to see the stretch sheet in an environment of 25°C and 40% RH and scored the feel in accordance with the following scale. The scores assigned by the 10 panelists were averaged to give a feel value. The average was rounded to the nearest whole number.
5=Good; 4=fairly good; 3=medium; 2=slightly poor; 1=poor
Table 4

| | | Example 3-1 | Example 3-2 | Example 3-3 | Example 3-4 |
|---|---|---|---|---|---|
| Stretch Sheet Production Conditions | Diameter ($\mu$m) of spinning nozzle | 400 | 400 | 400 | 400 |
| | Pitch (mm) of spinning nozzles | 1 | 1 | 1 | 1 |
| | Draw ratio of elastic filaments | 14 | 14 | 14 | 14 |
| | Travelling distance L (mm) from tip of spinning nozzle to joining point | 100 | 100 | 100 | 100 |
| | Clearance W (mm) of nip rollers | 0.2 | 0.05 | 0.5 | 1 |
| Properties of Elastic Filaments and Nonwoven Fabrics before Joining | Thickness TF ($\mu$m) of elastic filament | 107 | 107 | 107 | 107 |
| | Thickness TN1 (mm) of 1st nonwoven fabric | 0.27 | 0.27 | 0.27 | 0.27 |
| | Thickness TN2 (mm) of 2nd nonwoven fabric | 0.27 | 0.27 | 0.27 | 0.27 |
| | Thickness ratio W/ (TF+TN1+TN2) | 0.31 | 0.08 | 0.77 | 1.55 |

(continued)

|  |  | Example 3-1 | Example 3-2 | Example 3-3 | Example 3-4 |
|---|---|---|---|---|---|
| Stretch Sheet | 1st Nonwoven fabric basis weight (g/m$^2$) | 20 | 20 | 20 | 20 |
|  | 2nd Nonwoven fabric basis weight (g/m$^2$) | 20 | 20 | 20 | 20 |
|  | Elastic filaments weight per unit area (g/m$^2$) | 8 | 8 | 8 | 8 |
|  | Stretch sheet basis weight (g/m$^2$) | 48 | 48 | 48 | 48 |
|  | Average diameter ($\mu$m) of elastic filaments | 107 | 107 | 107 | 107 |
|  | Diameter ($\mu$m) of elastic filaments in planar direction | 128 | 173 | 121 | 117 |
|  | Diameter ($\mu$m) of elastic filaments in thickness direction | 86 | 41 | 93 | 97 |
|  | Elastic filament diameter in thickness direction/ stretch sheet thickness (%) | 6.6 | 3.7 | 7.2 | 6.9 |
|  | Average flatness of elastic filaments | 1.5 | 4.2 | 1.3 | 1.2 |
|  | Average bonding ratio (%) between elastic filaments and nonwoven fibers | 39 | 51 | 23 | 16 |
|  | 25% Retraction strength/ 25% extension strength (%) | 55 | 53 | 57 | 61 |
|  | 25% Retraction strength (cN/50 mm) | 35 | 30 | 42 | 47 |
|  | 25% Retraction strength (cN/{50 mm·(g/m$^2$)}) | 4.4 | 3.8 | 5.3 | 5.9 |
|  | Stretch sheet thickness (mm) | 1.3 | 1.1 | 1.3 | 1.4 |
|  | 1st Nonwoven fabric thickness (mm) | 0.6 | 0.5 | 0.6 | 0.6 |
|  | 2nd Nonwoven fabric thickness (mm) | 0.6 | 0.5 | 0.6 | 0.6 |
|  | Feel of stretch sheet | 5 | 4 | 5 | 5 |
|  | Interlaminar peel strength (cN/25 mm) | 50 | 80 | 35 | 21 |
|  | Width contraction (%) | 4 | 4 | 4 | 4 |
|  | Intersection of elastic filaments | few | few | few | few |

Table 5

| | | Example 3-5 | Example 3-6 | Example 3-7 | Comparative Example 3-1 |
|---|---|---|---|---|---|
| Stretch Sheet Production Conditions | Diameter ($\mu$m) of spinning nozzle | 400 | 400 | 400 | - |
| | Pitch (mm) of spinning nozzles | 1 | 1 | 1 | - |
| | Draw ratio of elastic filaments | 14 | 14 | 14 | - |
| | Travelling distance L (mm) from tip of spinning nozzle to joining point | 100 | 200 | 600 | 100 |
| | Clearance W (mm) of nip rollers | 0[*1] | 0[*1] | 0[*1] | 0[*1] |
| Properties of Elastic Filaments and Nonwoven Fabrics before Joining | Thickness TF ($\mu$m) of elastic filament | 107 | 107 | 107 | - |
| | Thickness TN 1 (mm) of 1st nonwoven fabric | 0.27 | 0.27 | 0.27 | 0.27 |
| | Thickness TN2 (mm) of 2nd nonwoven fabric | 0.27 | 0.27 | 0.27 | 0.27 |
| | Thickness ratio W/ (TF+TN1+TN2) | 0.00 | 0.00 | 0.00 | - |
| | 1st Nonwoven fabric basis weight (g/m$^2$) | 20 | 20 | 20 | 20 |
| | 2nd Nonwoven fabric basis weight (g/m$^2$) | 20 | 20 | 20 | 20 |
| | Elastic filaments weight per unit area (g/m$^2$) | 8 | 8 | 8 | 35 |
| | Stretch sheet basis weight (g/m$^2$) | 38 | 38 | 38 | 75 |
| | Average diameter ($\mu$m) of elastic filaments | 107 | 107 | 107 | - |
| | Diameter ($\mu$m) of elastic filaments in planar direction | 187 | 161 | 170 | - |
| | Diameter ($\mu$m) of elastic filaments in thickness direction | 27 | 54 | 44 | - |

(continued)

| | | Example 3-5 | Example 3-6 | Example 3-7 | Comparative Example 3-1 |
|---|---|---|---|---|---|
| Stretch Sheet | Elastic filament diameter in thickness direction/ stretch sheet thickness (%) | 2.7 | 4.5 | 3.6 | - |
| | Average flatness of elastic filaments | 6.9 | 3.0 | 3.9 | - |
| | Average bonding ratio (%) between elastic filaments and nonwoven fibers | 54 | 44 | 35 | - |
| | 25% Retraction strength/25% extension strength (%) | 41 | 54 | 56 | 31 |
| | 25% Retraction strength (cN/50 mm) | 18 | 33 | 36 | 31 |
| | 25% Retraction strength (cN/{50 mm·(g/m$^2$)}) | 2.3 | 4.1 | 4.5 | 0.9 |
| | Stretch sheet thickness (mm) | 1 | 1.2 | 1.2 | 0.9 |
| | 1st Nonwoven fabric thickness (mm) | 0.5 | 0.6 | 0.6 | 0.3 |
| | 2nd Nonwoven fabric thickness (mm) | 0.5 | 0.6 | 0.6 | 0.3 |
| | Feel of stretch sheet | 4 | 5 | 5 | 2 |
| | Interlaminar peel strength (cN/25 mm) | 123 | 82 | 34 | 146 |
| | Width contraction (%) | 4 | 4 | 4 | 28 |
| | Intersection of elastic filaments | few | few | none[*2] | - |
| *1: Contacting over entire width direction. *2: Overlapping was observed in part. | | | | | |

The results in Tables 4 and 5 apparently prove that the stretch sheets of Examples have a desired average bonding ratio, a high interlaminar peel strength (bonding strength between elastic filaments and nonwoven fabrics), a high 25% retraction strength (25% retraction strength per unit elastic resin content in stretch sheet), a small width contraction, few intersections of the elastic filaments, and excellent feel. In addition, the stretch sheets of Examples have a good appearance as illustrated in Fig. 2. In contrast, the stretch sheet of Comparative Example 3-1 has a low 25% retraction strength per unit elastic resin content in the stretch sheet, a large width contraction, and no breathability.

Industrial Applicability

Since the stretch sheet of the invention has the elastic filaments arranged to extend in the same direction, it stretches in the extending direction of the elastic filaments without undergoing contraction in width. The stretch sheet of the invention has a high strength so that peeling between the elastic filaments and the nonwoven fabric hardly occurs when stretched.

When the stretch sheet of the invention has a number of elastic filaments arranged to extend in the same direction on an extensible nonwoven fabric containing inelastic fibers and satisfies the specific conditions in terms of bonding strength between the elastic filaments and the nonwoven fabric, 25% retraction strength per unit elastic resin content in the stretch sheet, and average bonding ratio between the elastic filaments and the fibers of the nonwoven fabric, the stretch sheet has a good balance between interlaminar bonding strength and stretch characteristics such that it stretches in the extending direction of the elastic filaments without causing contraction in width and that separation between the elastic filaments and the nonwoven fabric hardly occurs when stretched.

According to the invention, excellent spinnability into elastic filaments is achieved to enable high speed production. That is, elastic filaments are spun from spinning nozzles without breakage with even a small diameter and without vibration. When the stretch sheet containing such elastic filaments is used as a material making a variety of garments exemplified by absorbent articles, such as disposable diapers, and other various articles applied to or worn by human bodies, it provides a snug fit and effectively prevents the garment from sliding down. The absorbent article having the stretch sheet of the invention achieves a good fit and is prevented from sliding down while being worn.

**Claims**

1. A stretch sheet comprising an extensible nonwoven fabric and a plurality of elastic filaments, the elastic filaments being arranged to extend in one direction without intersecting with each other and bonded to the nonwoven fabric in their substantially nonstretched state over their whole length.

2. The stretch sheet according to claim 1, wherein the elastic filaments are fusion bonded to the nonwoven fabric.

3. The stretch sheet according to claim 1 or 2, wherein the elastic filaments are formed by drawing an elastic resin in a molten state or a softened state.

4. The stretch sheet according to claim 3, wherein the elastic filaments are obtained by melt-drawing an elastic resin spun from spinning nozzles.

5. The stretch sheet according to claim 3 or 4, wherein the elastic filaments are obtained by drawing the elastic resin in a molten state or softened state at a draw ratio of 1.1 to 400, and have a diameter of 10 to 200 $\mu$m.

6. The stretch sheet according to any one of claims 1 to 5, having two or more zones different in extension stress in the extending direction of the elastic filaments, the two or more zones being in a substantially parallel relationship with respect to the extending direction of the elastic filaments and being different from each other in a distance between the elastic filaments and/or a diameter of the elastic filaments.

7. The stretch sheet according to any one of claims 1 to 6, wherein the nonwoven fabric comprises the same or different two nonwoven fabrics, and the elastic filaments are held between the two nonwoven fabrics.

8. The stretch sheet according to any one of claims 1 to 7, wherein the elastic filaments have an average flatness of 1.0 to 7.0.

9. The stretch sheet according to any one of claims 1 to 8, wherein the extensible nonwoven fabric contains inelastic fibers having a varied thickness along their length.

10. The stretch sheet according to claim 9, wherein the inelastic fibers have a thickness of 2 to 15 $\mu$m at their finest portion and of 10 to 40 $\mu$m at their thickest portion.

11. The stretch sheet according to claim 9 or 10, wherein the inelastic fibers are staple fibers comprising conjugate fibers.

12. The stretch sheet according to any one of claims 1 to 11, having a bonding strength between the elastic filaments and the nonwoven fabric of 10 cN/25 mm or more, a 25% retraction strength, per unit basis weight of an elastic resin present in the stretch sheet, of 1.0 cN/ {50 mm·(g/m$^2$)} or more, and an average bonding ratio between the elastic filaments and fibers of the nonwoven fabric of 10% to 60%.

**13.** The stretch sheet according to any one of claims 1 to 12, wherein the elastic filaments comprise a resin composition comprising an A1-B-A2 triblock copolymer containing polymer blocks A1 and A2 each composed mainly of an aromatic vinyl polymer and a polymer block B composed mainly of a polyolefin, a sum of a weight average molecular weight of the polymer block A1 and that of the polymer block A2 as measured by gel permeation chromatography being 14000 to 20000, and a weight average molecular weight of the polymer block B as measured by gel permeation chromatography being 40000 to 80000.

**14.** The stretch sheet according to claim 13, wherein the resin composition has a maximum melt tension of 0.06 to 0.20 cN when the resin composition is extruded in a molten state at 245°C from an orifice of a cylindrical nozzle of 0.45 mm in inner diameter and 4.5 mm in length at an extrusion rate of 8.4 m/min.

**15.** A process of producing a stretch sheet comprising the steps of:

taking off a plurality of molten elastic filaments as spun from spinning nozzles at a predetermined speed while drawing the filaments, fusion bonding the elastic filaments to a nonwoven fabric before the filaments solidify such that the filaments extend in one direction in a non-intersection relation to each other to make a composite, and stretching the composite in the extending direction of the elastic filaments to impart stretchability to the composite.

**16.** The process of producing a stretch sheet according to claim 15, wherein the step of taking off the elastic filaments while drawing is carried out by fusion bonding the elastic filaments to the moving nonwoven fabric.

**17.** The process of producing a stretch sheet according to claim 15 or 16, wherein the step of stretching is carried out by passing the composite in a moving direction of the composite between a pair of corrugating rollers each having alternating ridges and grooves along the circumferential direction thereof, thereby imparting stretchability to the composite in the moving direction of the composite.

**18.** A process of producing a stretch sheet according to claim 15, wherein the distance of travel of the elastic filaments from the tip of the spinning nozzles until contact with the nonwoven fabric is 600 mm or shorter.

**19.** The process of producing a stretch sheet according to claim 18, wherein the step of fusion bonding the elastic filaments to the nonwoven fabric is carried out by joining the moving nonwoven fabric and the elastic filaments in a molten state as spun from the spinning nozzles by introducing the nonwoven fabric and the elastic filaments between a pair of nip rollers, with a clearance of the rollers being set to 0.05 to 1.0 mm.

**Patentansprüche**

**1.** Dehnbare Folie mit einem streckbaren Vlies und mehreren elastischen Filamenten, wobei die elastischen Filamente so angeordnet sind, dass sie sich in eine Richtung erstrecken, ohne sich zu kreuzen, und in ihrem im Wesentlichen nichtgedehnten Zustand über ihre gesamte Länge mit dem Vlies verbunden sind.

**2.** Dehnbare Folie nach Anspruch 1, wobei die elastischen Filamente mit dem Vlies verschmolzen sind.

**3.** Dehnbare Folie nach Anspruch 1 oder 2, wobei die elastischen Filamente durch Ziehen eines elastischen Harzes in einem geschmolzenen Zustand oder einem erweichten Zustand gebildet wurden.

**4.** Dehnbare Folie nach Anspruch 3, wobei die elastischen Filamente durch Schmelzziehen eines aus Spinndüsen gesponnenen elastischen Harzes gewonnen wurden.

**5.** Dehnbare Folie nach Anspruch 3 oder 4, wobei die elastischen Filamente durch Ziehen des elastischen Harzes in einem geschmolzenen Zustand oder einem erweichten Zustand mit einem Ziehverhältnis von 1,1 bis 400 gewonnen wurden und einen Durchmesser von 10 bis 200$\mu$m haben.

**6.** Dehnbare Folie nach einem der Ansprüche 1 bis 5, die zwei oder mehr Zonen mit unterschiedlichen Streckspannungen in Streckrichtung der elastischen Filamente aufweist, wobei die zwei oder mehr Zonen in einer im Wesentlichen parallelen Beziehung bezüglich der Streckrichtung der elastischen Filamente sind und sich bezüglich eines Abstands zwischen den elastischen Filamenten und/oder eines Durchmessers der elastischen Filamente unterscheiden.

7. Dehnbare Folie nach einem der Ansprüche 1 bis 6, wobei das Vlies zwei gleiche Vliese oder zwei unterschiedliche Vliese aufweist und wobei die elastischen Filamente zwischen den zwei Vliesen gehalten werden.

8. Dehnbare Folie nach einem der Ansprüche 1 bis 7, wobei die elastischen Filamente eine durchschnittliche Flachheit von 1,0 bis 7,0 haben.

9. Dehnbare Folie nach einem der Ansprüche 1 bis 8, wobei das streckbare Vlies unelastische Fasern aufweist, die entlang ihrer Länge variable Dicke haben.

10. Dehnbare Folie nach Anspruch 9, wobei die unelastischen Fasern eine Dicke von 2 bis 15$\mu$m an ihrem dünnsten Abschnitt und von 10 bis 40$\mu$m an ihrem dicksten Abschnitt haben.

11. Dehnbare Folie nach Anspruch 9 oder 10, wobei die unelastischen Fasern Zellstofffasern sind, die konjugierte Fasern aufweisen.

12. Dehnbare Folie nach einem der Ansprüche 1 bis 11, wobei eine Bindefestigkeit zwischen den elastischen Filamenten und dem Vlies 10cN/25mm oder mehr ist, eine 25%-Retraktionsfestigkeit pro Basisgewichtseinheit eines in der dehnbaren Folie vorhandenen Harzes 1, 0cN/ {50mm·(g/m$^2$)} oder mehr ist und ein durchschnittliches Bindeverhältnis zwischen den elastischen Filamenten und Fasern des Vlieses 10% bis 60% ist.

13. Dehnbare Folie nach einem der Ansprüche 1 bis 12, wobei die elastischen Filamente eine Harzzusammensetzung aufweisen, die ein A1-B-A2-Triblock-Copolymer aufweist, das Polymerblöcke A1 und A2, von denen jeder hauptsächlich aus einem aromatischen VinylPolymer besteht, und einen Polymerblock B enthält, der hauptsächlich aus einem Polyolefin besteht,
wobei eine Summe aus dem durchschnittlichen Molekulargewicht des Polymerblocks A1 und demjenigen des Polymerblocks A2, gemessen durch Gelpermeation-Chromatographie, 14000 bis 20000 ist und ein durchschnittliches Molekulargewicht des Polymerblocks B, gemessen durch Gelpermeation-Chromatographie, 40000 bis 80000 ist.

14. Dehnbare Folie nach Anspruch 13, wobei die Harzzusammensetzung eine maximale Schmelzspannung von 0,06 bis 0,20cN hat, wenn die Harzzusammensetzung in einem geschmolzenen Zustand bei 245°C aus einer Öffnung einer zylindrischen Düse von 0,45mm Innendurchmesser und 4,5mm Länge bei einer Extrusionsrate von 8,4m/min extrudiert wird.

15. Verfahren zur Herstellung einer dehnbaren Folie mit den folgenden Schritten:

Wegführen mehrerer aus Spinndüsen gesponnener geschmolzener elastischer Filamente bei einer vorgegebenen Geschwindigkeit unter gleichzeitigem Ziehen der Filamente,
Verschmelzen der elastischen Filamente mit einem Vlies vor Erstarren der Filamente, derart, dass sich die Filamente in eine Richtung erstrecken, ohne sich zu kreuzen, um einen Verbund zu erzeugen,
Dehnen des Verbunds in die Streckrichtung der elastischen Filamente, um dem Verbund eine Dehnbarkeit zu verleihen.

16. Verfahren zur Herstellung einer dehnbaren Folie nach Anspruch 15, wobei der Schritt des Wegführens der elastischen Filamente unter gleichzeitigem Ziehen durchgeführt wird durch Verschmelzen der elastischen Filamente mit dem sich bewegenden Vlies.

17. Verfahren zur Herstellung einer dehnbaren Folie nach Anspruch 15 oder 16, wobei der Dehnschritt durchgeführt wird, indem der Verbund in seiner Bewegungsrichtung zwischen zwei gerieften Walzen, von denen jede entlang ihres Umfangs abwechselnd Rippen und Rillen aufweist, hindurchgeführt wird, wodurch dem Verbund in seiner Bewegungsrichtung eine Dehnbarkeit verliehen wird.

18. Verfahren zur Herstellung einer dehnbaren Folie nach Anspruch 15, wobei die Wegstrecke der elastischen Filamente von der Spitze der Spinndüsen bis zum Kontakt mit dem Vlies 600mm oder kürzer ist.

19. Verfahren zur Herstellung einer dehnbaren Folie nach Anspruch 18, wobei der Schritt des Verschmelzens der elastischen Filamente mit dem Vlies durchgeführt wird, indem durch Einführen des Vlieses und der elastischen Filamente zwischen zwei Klemmwalzen, deren Spalt auf 0,05 bis 1,0mm eingestellt ist, das sich bewegende Vlies und die aus den Spinndüsen gesponnenen elastischen Filamente in einem geschmolzenen Zustand verbunden werden.

## Revendications

1. Feuille étirable comprenant un textile non tissé extensible et une pluralité de filaments élastiques, les filaments élastiques étant agencés pour s'étendre dans une direction sans se croiser les uns les autres et collés au textile non tissé dans leur état sensiblement non étiré sur toute leur longueur.

2. Feuille étirable selon la revendication 1, dans laquelle les filaments élastiques sont collés par fusion au textile non tissé.

3. Feuille étirable selon la revendication 1 ou 2, dans laquelle les filaments élastiques sont formés en étirant une résine élastique dans un état fondu ou dans un état ramolli.

4. Feuille étirable selon la revendication 3, dans laquelle les filaments élastiques sont obtenus en étirant dans un état fondu une résine élastique filée à partir de filières.

5. Feuille étirable selon la revendication 3 ou 4, dans laquelle les filaments élastiques sont obtenus en étirant la résine élastique dans un état fondu ou dans un état ramolli selon un rapport d'étirage de 1,1 à 400 et ont un diamètre de 10 à 200 $\mu$m.

6. Feuille étirable selon l'une quelconque des revendications 1 à 5, ayant deux zones ou plus ayant une contrainte d'extension différente dans la direction d'extension des filaments élastiques, les deux zones ou plus étant dans une relation sensiblement parallèle par rapport à la direction d'extension des filaments élastiques et différentes les unes des autres sur une distance comprise entre les filaments élastiques et/ou un diamètre des filaments élastiques.

7. Feuille étirable selon l'une quelconque des revendications 1 à 6, dans laquelle le textile non tissé comprend deux textiles non tissés identiques ou différents et les filaments élastiques sont maintenus entre les deux textiles non tissés.

8. Feuille étirable selon l'une quelconque des revendications 1 à 7, dans laquelle les filaments élastiques ont une planéité moyenne de 1,0 à 7,0.

9. Feuille étirable selon l'une quelconque des revendications 1 à 8, dans laquelle le textile non tissé extensible contient des fibres non élastiques ayant une épaisseur variée sur leur longueur.

10. Feuille étirable selon la revendication 9, dans laquelle les fibres non élastiques ont une épaisseur de 2 à 15 $\mu$m au niveau de leur partie la plus fine et de 10 à 40 $\mu$m au niveau de leur partie la plus épaisse.

11. Feuille étirable selon la revendication 9 ou 10, dans laquelle les fibres non élastiques sont des fibres coupées comprenant des fibres conjuguées.

12. Feuille étirable selon l'une quelconque des revendications 1 à 11, ayant un pouvoir d'adhérence entre les filaments élastiques et le textile non tissé de 10 cN/25 mm ou plus, une résistance à la rétraction de 25 %, par poids de base unitaire d'une résine élastique présente dans la feuille étirable, de 1, 0 cN/ {50 mm•(g/m$^2$)} ou plus, et un rapport d'adhérence moyen entre les filaments élastiques et les fibres du textile non tissé de 10 % à 60 %.

13. Feuille étirable selon l'une quelconque des revendications 1 à 12, dans laquelle les filaments élastiques comprennent une composition de résine comprenant un copolymère tribloc A1-B-A2 contenant les blocs polymères A1 et A2, chacun étant composé principalement d'un polymère vinylique aromatique et d'un bloc B polymère composé principalement d'une polyoléfine, la somme de la masse moléculaire moyenne en poids du bloc polymère A1 et de celle du bloc polymère A2 telles que mesurées par chromatographie d'exclusion étant de 14 000 à 20 000 et la masse moléculaire moyenne en poids du bloc polymère B telle que mesurée par chromatographie d'exclusion étant de 40 000 à 80 000.

**14.** Feuille étirable selon la revendication 13, dans laquelle la composition de résine a une tension maximale à l'état fondu de 0,06 à 0,20 cN lorsque la composition de résine est extrudée à l'état fondu à 245°C à partir d'un orifice de filière cylindrique de 0,45 mm de diamètre interne et de 4,5 mm de long à une vitesse d'extrusion de 8,4 m/min.

**15.** Procédé pour produire une feuille étirable comprenant les étapes consistant à :

retirer une pluralité de filaments élastiques fondus tels que filés à partir de filières à une vitesse prédéterminée tout en étirant les filaments,
coller par fusion les filaments élastiques à un textile non tissé avant que les filaments ne se solidifient, de telle sorte que les filaments s'étendent dans une direction sans se croiser les uns les autres pour créer un composite, et à
étirer le composite dans une direction d'extension des filaments élastiques pour conférer une étirabilité au composite.

**16.** Procédé pour produire une feuille étirable selon la revendication 15, dans lequel l'étape consistant à retirer les filaments élastiques pendant l'étirage est réalisée en collant par fusion les filaments élastiques au textile non tissé en mouvement.

**17.** Procédé pour produire une feuille étirable selon la revendication 15 ou 16, dans lequel l'étape d'étirage est réalisée en faisant passer le composite dans une direction de déplacement du composite entre une paire de cylindres cannelés ayant chacun des crêtes et des rainures alternées le long de leur direction circonférentielle, conférant ainsi une étirabilité au composite dans la direction de déplacement du composite.

**18.** Procédé pour produire une feuille étirable selon la revendication 15, dans lequel la distance de déplacement des filaments élastiques de la pointe des filières jusqu'au contact avec le textile non tissé est de 600 mm ou moins.

**19.** Procédé pour produire une feuille étirable selon la revendication 18, dans lequel l'étape consistant à coller par fusion les filaments élastiques au textile non tissé est réalisée en assemblant le textile non tissé en mouvement et les filaments élastiques à l'état fondu filés à partir des filières en introduisant le textile non tissé et les filaments élastiques entre une paire de rouleaux presseurs, l'espace entre les rouleaux étant réglé pour être de 0,05 à 1,0 mm.

Fig.1

Fig.2(a)

Fig.2(b)

## Fig. 3

Fine portion

Thick portion

20 μm

Fine portion

## Fig. 4

9.55mm

0.45mm

4.5mm

Extrusion rate

(8.4m/min)

Take-off

Load cell

Fig. 5

Fig. 6

Fig. 7

Machine direction

# Fig. 8

Inelastic fiber

Stretching → Bonds → Release from tension

# Fig. 9(a)   Fig. 9(b)

Fig. 9(a): Clip, Diaper, Initial position A, Acrylic resin pipe, 50mm, Jack

Fig. 9(b): Displacement position, Initial position A

**EP 2 098 363 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0020206 A1
- WO 0020207 A1
- WO 9534264 A1
- WO 0187214 A1
- US 6730390 B1
- JP 2004218183 A